(19) Europäisches Patentamt
European Patent Office
Office européen des brevets

(11) EP 0 628 542 B1

(12) EUROPÄISCHE PATENTSCHRIFT

(45) Veröffentlichungstag und Bekanntmachung des Hinweises auf die Patenterteilung:
**09.04.1997 Patentblatt 1997/15**

(51) Int Cl.[6]: **C07C 269/04**

(21) Anmeldenummer: **94108041.8**

(22) Anmeldetag: **25.05.1994**

# (54) Verfahren zur Herstellung von organischen Carbamaten

Process for the preparation of organic carbamates

Procédé de préparation de carbamates organiques

(84) Benannte Vertragsstaaten:
**AT BE CH DE DK ES FR GB IT LI NL PT SE**

(30) Priorität: **07.06.1993 DE 4318889**

(43) Veröffentlichungstag der Anmeldung:
**14.12.1994 Patentblatt 1994/50**

(73) Patentinhaber: **BAYER AG**
**51368 Leverkusen (DE)**

(72) Erfinder: **Heuer, Lutz, Dr.**
**D-47800 Krefeld (DE)**

(56) Entgegenhaltungen:
**EP-A- 0 511 948**

- **CHEMICAL ABSTRACTS, vol. 111, no. 19, 1989, Columbus, Ohio, US; abstract no. 173191b, Y. YOSHIDA ET AL**
- **TETRAHEDRON, Bd.48, Nr.8, 1992, Seiten 1515 - 1530, M. ARESTA ET AL**
- **CHEMICAL ABSTRACTS, vol. 106, no. 15, 1987, Columbus, Ohio, US; abstract no. 119278u, S. ISHII ET AL**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 628 542 B1

**Beschreibung**

Die vorliegende Erfindung betrifft ein besonders vorteilhaftes und einfach durchzuführendes Verfahren zur Herstellung von organischen Carbamaten aus Aminen, Kohlendioxid und einem Alkylierungsmittel.

Es ist bekannt, daß man Diethylamin mit Alkylhalogeniden unter $CO_2$-Druck zu Carbamaten umsetzen kann (siehe z.B. Chem. Letters 1984, 1571-1572). Geeignete Reaktionsbedingungen sind beispielsweise 40 atm Druck, 70°C und 48 Stunden Reaktionszeit. Die Ausbeuten liegen im Bereich 6 bis 53 %. Nachteilig sind der Aufwand für das Arbeiten unter Druck, die langen Reaktionszeiten und die häufig nur geringen bis sehr geringen Ausbeuten. In Bull. Chem. Soc. Jap. 62, 1534 (1989) wird ausgeführt, daß bei dieser Arbeitsweise mehr als 5 Gew.-% Lösungsmitel (etwa Dimethylformamid oder Dimethylsulfoxid) zu Ausbeuteverlusten führen.

Die Umsetzung eines schwach sauer reagierenden Amins (= 2-Alkylindol) mit n-Butyllithium und Kohlendioxid führt zum entsprechenden Lithiumcarbamat, das mit Alkylierungsmitteln in Gegenwart einer starken organischen Base (= t-Butyllithium) im Aminteil, jedoch nicht an der Carbamatgruppe alkyliert werden kann (siehe z.B. J. Am. Chem. Soc. 108, 6808-6809 (1986)). Es handelt sich hier demnach um kein Verfahren zur Herstellung organischer Carbamate, sondern um eine Alkylierung im Aminteil von Carbamaten.

Andere $\alpha,\beta$-ungesättigte, d.h. schwach sauer reagierende (= elektrophile) Amine, deren Metallsalze und die daraus zugänglichen Metallcarbamate lassen sich katalysatorfrei oder mit basischen Katalysatoren zur organischen Carbamaten umsetzen (siehe z.B. US-A 3 147 262, US-A 3 299 076, FR-A 2 444 030 und J. Org. Chem. 54, 2425 (1989)).

Der Fachmann schließt eine Übertragbarkeit von solchen Reaktionen von schwach sauer reagierenden Aminen auf typische basisch reagierende und nukleophile Amine aus, da er dann unter Berücksichtigung von J. Am. Chem. Soc. 108, 6808-6809 (1986) (siehe oben) und J. Chem. Soc. Chem. Commun., 1979, 797 Alkylierungen im Aminteil und nicht an der Carbamatgruppe erwartet.

Es gibt auch Verfahren zur Herstellung von organischen Carbamaten aus Aminen, Kohlendioxid und Alkylierungsmitteln oder aus Metallcarbamaten und Alkylierungsmitteln, bei denen wenig umweltfreundliche und/oder teure Verbindungen eingesetzt werden müssen, wie Silbercarbamate, Zinkalkyle, Palladiumverbindungen und Kronenether (siehe z.B. JP-OS 49-81371, JP-OS 51-113852, Bull. Chem. Soc. Japan 61, 2613 (1988), J. Chem. Soc. Dalton Trans. 1989, 1007 und EP-A 477 159). Solche Verfahren sind für eine gewerbliche Anwendung wenig geeignet.

Schließlich ist es auch bekannt geworden, organische Carbonate aus Aminen, Kohlendioxid und Alkylhalogeniden in Gegenwart stöchiometrischer oder überschüssiger Mengen starker organischer Basen, z.B. Diazabicyclo(5.4.0)-undec-7-en (= DBU), herzustellen (siehe z.B. EP-A 511 948, Chemistry Express 1, 224 (1986)). Die erforderlichen großen Mengen an aufwendig herzustellenden, schwierig abtrennbaren und schwierig zu entsorgenden organischen Basen, die Verwendung spezieller Lösungs mittel und der weitgehende Ausschluß von Wasser machen dieses Verfahren für eine gewerbliche Verwendung ebenfalls uninteressant. Außerdem neigen starke Basen, z.B. DBU, zu einer Vielzahl von Reaktionen, z.B. Eliminierungen, was die Wahrscheinlichkeit der Bildung von Nebenprodukten erhöht (Oediger et al., Synthesis 1972, 591).

Speziell in der EP-A 511 948 ist noch angegeben, daß man nur mit großen Überschüssen an Alkylhalogeniden gute Ausbeuten erhält. Das ist natürlich wirtschaftlich betrachtet sehr unvorteilhaft.

Die katalytisch forcierte Addition von Alkenen, Alkinen, Kohlendioxid und Aminen zu Carbamaten ist ebenfalls bekannt (J. Molecular Catalysis 74, 97 (1992)). Hierbei werden allerdings die verwendeten Alkylierungsmittel in ihrer Funktionalität verändert, was erfindungsgemäß nicht der Fall ist.

Es wurde nun ein Verfahren zur Herstellung von organischen Carbamaten aus einem basisch reagierenden Amin, Kohlendioxid und einem Alkylierungsmittel gefunden, das dadurch gekennzeichnet ist, daß man zunächst das Amin mit Kohlendioxid in Gegenwart von einer oder mehreren basischen Verbindungen der Elemente Lithium, Natrium, Magnesium, Kalium, Kalzium, Rubidium, Strontium, Cäsium, Barium und/oder des Ammoniums zur Reaktion bringt und dann das Alkylierungsmittel hinzufügt.

In das erfindungsgemäße Verfahren können die verschiedensten basisch reagierenden Amine eingesetzt werden, beispielsweise solche der Formel (I)

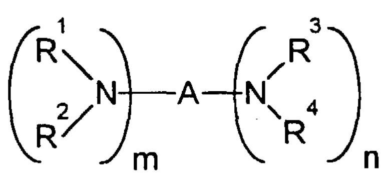

(I),

in der

$R^1$, $R^2$, $R^3$ und $R^4$ gleich oder verschieden sind und jeweils für Wasserstoff oder einen der folgenden Reste in einbindiger Form stehen: $C_1$-$C_{30}$-Alkyl, $C_3$-$C_{30}$-Alkenyl, $C_3$-$C_{30}$-Alkinyl, $C_3$-$C_{12}$-Cycloalkyl, $C_5$-$C_{12}$-Cycloalkenyl, $C_8$-$C_{12}$-Cycloalkinyl, $C_6$-$C_{14}$-Aryl, $C_5$-$C_{13}$-Hetaryl mit bis zu 3 Sauerstoff-, Schwefel- und/oder Stickstoffatomen im Ringsystem, $C_7$-$C_{20}$-Aralkyl, $C_9$-$C_{20}$-Aralkenyl, $C_9$-$C_{20}$-Aralkinyl, $C_7$-$C_{20}$-Alkaryl, $C_8$-$C_{20}$-Alkenaryl oder $C_8$-$C_{20}$-Alkinaryl,

die gegebenenfalls ein- bis fünfmal durch O-$C_1$-$C_{12}$-Alkyl oder -$C_6$-$C_{10}$-Aryl, $NH_2$, NH-$C_1$-$C_{12}$-Alkyl oder -$C_6$-$C_{10}$-Aryl, N($C_1$-$C_{12}$-Alkyl oder -$C_6$-$C_{10}$-Aryl)$_2$, COO-$C_1$-$C_{12}$-Alkyl oder COO-$C_6$-$C_{10}$-Aryl, $CONH_2$, CONH-$C_1$-$C_{12}$-Alkyl, CON($C_1$-$C_{12}$-Alkyl)$_2$, Halogen, OP(O-$C_1$-$C_{12}$-Alkyl oder -$C_6$-$C_{10}$-Aryl)$_2$, Si($C_1$-$C_{12}$-Alkyl und/oder $C_6$-$C_{10}$-Aryl)$_3$, Si (O-$C_1$-$C_{12}$-Alkyl und/oder $C_6$-$C_{10}$-Aryl)$_3$, Si($C_1$-$C_{12}$-Alkyl oder -$C_6$-$C_{10}$-Aryl)(O-$C_1$-$C_{12}$-Alkyl und/oder O-$C_6$-$C_{10}$-Aryl)$_2$, Si($C_1$-$C_{12}$-Alkyl und/oder $C_6$-$C_{10}$-Aryl)$_2$(O-$C_1$-$C_{12}$-Alkyl oder O-$C_6$-$C_{10}$-Aryl), OS($C_1$-$C_{12}$-Alkyl oder $C_6$-$C_{10}$-Aryl, $O_2$S($C_1$-$C_{12}$-Alkyl oder $C_6$-$C_{10}$-Aryl), CHO, CN, C(O-$C_1$-$C_{12}$-Alkyl oder -$C_6$-$C_{10}$-Aryl)$_2$, OC($C_1$-$C_{12}$-Alkyl oder $C_6$-$C_{10}$-Aryl), $NO_2$, $CF_3$, $OCF_3$, $OCF_2H$, $OCFH_2$, $OCF_2CF_3$, $OCH_2CF_3$, OCO($C_1$-$C_{12}$-Alkyl oder $C_6$-$C_{10}$-Aryl), HN-CO($C_1$-$C_{12}$-Alkyl oder $C_6$-$C_{10}$-Aryl)$_3$, OP($C_1$-$C_{12}$-Alkyl oder $C_6$-$C_{10}$-Aryl)$_3$, OP(O-$C_1$-$C_{10}$-Alkyl oder -$C_6$-$C_{10}$-Aryl)$_2$ ($C_1$-$C_{12}$-Alkyl oder $C_6$-$C_{10}$-Aryl) substituiert sein können

und/oder gegebenenfalls durch Sauerstoff- oder Schwefelatome oder N($C_1$-$C_{12}$-Alkyl oder $C_6$-$C_{10}$-Aryl)-Gruppen unterbrochen sein können,

wobei auch $R^1$ und $R^2$ oder $R^3$ und $R^4$ jeweils gemeinsam einen der zuvor definierten Reste, dann allerdings in zweibindiger Form darstellen können,

oder $R^3$ und $R^4$ gemeinsam einen 3- bis 10-gliedrigen Alkylring darstellen können, der gegebenenfalls mit einer bis zwei $C_1$-$C_{10}$-Alkylgruppen substituiert und/oder gegebenenfalls durch ein oder zwei Sauerstoff-, Schwefel- und/oder Stickstoffatomen unterbrochen sein kann,

A für Wasserstoff oder einen wie bei $R^1$ definierten Rest, jedoch in zweibindiger Form steht,

m für Null oder eine ganze Zahl von 1 bis 10 steht und

n für eine ganze Zahl von 1 bis 10 steht,

mit den Maßgaben, daß

a) $R^1$, $R^2$, $R^3$ und $R^4$ nicht für solche Reste stehen, die eine isolierte $\alpha,\beta$-Mehrfachbindung enthalten,

b) im Falle von $m \neq$ Null, wenigstens einer der Reste $R^1$, $R^2$, $R^3$ und $R^4$ für Wasserstoff steht,

c) im Falle A = Wasserstoff, m für Null steht und

d) im Falle A = Wasserstoff, $R^1$ und $R^2$ nicht gleichzeitig auch für Wasserstoff stehen.

Die Maßgabe a) schließt im wesentlichen En-Amine aus.
Halogen kann z.B. Fluor, Chlor, Brom oder Jod bedeuten.

Einzelne, nicht erschöpfend aufgezählte Beispiele für Alkylgruppen, auch in zusammengesetzten Resten, sind Methyl, Ethyl, Propyl, Butyl, Pentyl, Hexyl, Heptyl, Octyl, Nonyl, Decyl, Undecyl, Dodecyl, Tridecyl, Tetradecyl, Pentadecyl und Hexadecyl, die jeweils geradkettig oder verzweigt und/oder gegebenenfalls mit Fluor substituiert sein können.

Einzelne, nicht erschöpfend aufgezählte Beispiele für Alkoxygruppen, auch in zusammengesetzten Resten sind Methoxy, Ethoxy, Propyloxy, Butyloxy, Pentyloxy, Hexyloxy, Heptyloxy, Octyloxy, Nonyloxy, Decyloxy, Undecyloxy und Dedecyloxy, die jeweils geradkettig oder verzweigt und/oder gegebenenfalls mit Fluor substituiert sein können.

Einzelne, nicht erschöpfend aufgezählte Beispiele für Hetarylgruppen sind Pyrazolyl, Imidazolyl, 1,2,4-Triazolyl, Pyrrolyl, Furanyl, Thienyl, Thiazolyl, Oxazolyl, Pyridinyl, Pyrimidinyl, Triazinyl, Chinolinyl, Isochinolinyl, Chinazolinyl, Indolyl, Benzothienyl, Benzofuranyl, Benzothiazolyl, Benzimidazolyl, Pyrazolylmethyl, Imidazolylmethyl, 1,2,4-Triazolylmethyl, Pyrrolylmethyl, Furfuryl, Thienylmethyl, Thiazolylmethyl, Oxazolylmethyl, Pyridinylmethyl, Pyrimidinylmethyl, Triazinylmethyl, Chinolinylmethyl, Isochinolinylmethyl, Chinazolinylmethyl, Indolylmethyl, Benzothienylmethyl, Benzofurfuryl, Benzothiazolylmethyl oder Benzimidazolylmethyl, wobei gegebenenfalls jede dieser Gruppen einfach bis dreifach, gleichartig oder verschieden substituiert sein kann, z.B. durch Fluor, Chlor, Brom, Methyl, Ethyl und/oder tert.-Butyl.

Man kann in das erfindungsgemäße Verfahren auch Polyamine einsetzen, die z.B. 21 bis 500.000

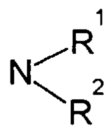 -Gruppen

enthalten, wobei bei wenigstens einer dieser Gruppen $R^1$ für Wasserstoff steht und $R^1$ und $R^2$ sonst die bei Formel (I) angegebene Bedeutung haben, wobei jedoch $R^1$ und $R^2$ nicht gemeinsam einen zweibindigen Rest darstellen können. Solche Polyamine können beispielsweise Molekulargewichte von 500 bis 3.500.000 aufweisen.

Bevorzugt sind Amine der Formel (I), bei denen $R^1$, $R^2$, $R^3$ und $R^4$ gleich oder verschieden sind und jeweils für Wasserstoff oder einen der folgenden Reste in einbindiger Form stehen:

$C_1$-$C_{14}$-Alkyl, $C_3$-$C_{10}$-Alkenyl, $C_3$-$C_{10}$-Alkinyl, $C_3$-$C_{12}$-Cycloalkyl, $C_5$-$C_7$-Cycloalkenyl, $C_6$-$C_{10}$-Aryl, $C_5$-$C_{10}$-Hetaryl mit bis zu 3 Sauerstoff-, Schwefel- und/oder Stickstoffatomen im Ringsystem, $C_7$-$C_{12}$-Aralkyl oder $C_7$-$C_{10}$-Alkaryl,

die gegebenenfalls ein- bis fünfmal durch O-$C_1$-$C_6$-Alkyl oder -$C_6$-$C_{10}$-Aryl, $NH_2$, NH-$C_1$-$C_6$-Alkyl oder -$C_6$-$C_{10}$-Aryl, N($C_1$-$C_6$-Alkyl oder $C_6$-$C_{10}$-Aryl)$_2$, COO-$C_1$-$C_6$-Alkyl, COO-$C_6$-$C_{10}$-Aryl, CONH-$C_1$-$C_6$-Alkyl, CON($C_1$-$C_6$-Alkyl)$_2$, OS($C_1$-$C_6$-Alkyl oder Phenyl), $O_2$S($C_1$-$C_6$-Alkyl oder Phenyl), CHO, CN, OC($C_1$-$C_6$-Alkyl oder Phenyl), $NO_2$, $CF_3$, $OCF_3$, $OCF_2H$, $OCFH_2$, OCO($C_1$-$C_6$-Alkyl oder Phenyl) oder HNCO($C_1$-$C_6$-Alkyl oder Phenyl substituiert sein können,

und/oder gegebenenfalls durch Sauerstoff- oder Schwefelatome oder N($C_1$-$C_6$-Alkyl oder Phenyl)-Gruppen unterbrochen sein können,

wobei auch $R^1$ und $R^2$ oder $R^3$ und $R^4$ jeweils gemeinsam einen der zuvor definierten Reste, dann allerdings in zweibindiger Form darstellen können,

oder $R^3$ und $R^4$ gemeinsam einen 5- bis 7-gliedrigen Alkylring darstellen können, der gegebenenfalls mit einer oder zwei $C_1$-$C_6$-Alkylgruppen substituiert ist und/oder gegebenenfalls durch ein Sauerstoff- oder Schwefelatom oder eine N($C_1$-$C_6$-Alkyl oder Phenyl)-Gruppe unterbrochen sein kann,

A für Wasserstoff oder einen der bei $R^1$ als bevorzugt definierten Reste in zweibindiger Form steht,

m für Null oder eine ganze Zahl von 1 bis 5 steht und

n für eine ganze Zahl von 1 bis 5 steht,

mit den Maßgaben, daß

a') $R^1$, $R^2$, $R^3$ und $R^4$ nicht für solche Reste stehen, die eine isolierte $\alpha,\beta$-Mehrfachbindung enthalten,

b') im Falle $m \neq$ Null wenigstens einer der Reste $R^1$, $R^2$, $R^3$ und $R^4$ für Wasserstoff steht,

c') im Falle A = Wasserstoff m für Null steht und

d') im Falle A = Wasserstoff $R^1$ und $R^2$ nicht gleichzeitig auch für Wasserstoff stehen.

Besonders bevorzugt sind Amine der Formel (I), bei denen $R^1$, $R^2$, $R^3$ und $R^4$ gleich oder verschieden sind und jeweils für Wasserstoff oder einen der folgenden Reste in einbindiger Form stehen:

Methyl, Ethyl, n-Propyl, i-Propyl, Butyl, Cyclohexyl, Phenyl, Pyrazolyl, Imidazolyl, Pyrrolyl, Furanyl, Thienyl, Pyridinyl, Chinolinyl, Isochinolinyl, Chinazolyl, Indolyl, Benzyl, Tolyl und Xylyl,

die gegebenenfalls ein- bis zweimal durch Methoxy, Ethoxy, Phenyl, CHO, CN, $OCF_3$, $OCF_2H$, Carboxymethyl, Carboxyethyl oder $NO_2$ substituiert sein können,

die gegebenenfalls ein- oder zweimal durch ein Sauerstoffatom oder eine N-Methyl-, N-Ethyl- oder N-Phenyl-Gruppe unterbrochen sein können,

wobei auch $R^1$ und $R^2$ gemeinsam einen der zuvor definierten besonders bevorzugten Reste, allerdings in zweibindiger Form darstellen können,

A für Wasserstoff oder einen wie bei $R^1$ als besonders bevorzugt definierten Rest, allerdings in zweibindiger Form steht,

m für Null, 1 oder 2 steht und

n für 1 oder 2 steht,

mit den Maßgaben wie oben bei den bevorzugten Amine angegeben.

Im einzelnen seien folgende Amine genannt: Dimethylamin, Diethylamin, Di-n-propylamin, Di-i-propylamin, Di-n-butylamin, Di-i-butylamin, Di-s-butylamin, Di-t-butylamin, Di-n-pentylamin, Diamylamin, Di-i-amylamin, Dihexylamin, Diheptylamin, Dioctylamin, Dinonylamin, Didecylamin, Diundecylamin, Didodecylamin, Ditridecylamin, Ditetradecylamin, Dipentadecylamin, Dihexadecylamin, Diheptadecylamin, Dioctadecylamin, Dicyclohexylamin, Dibenzylamin, Diphenylamin, Diallylamin, N-Methylanilin, N-Ethylanilin, N-Propylanilin, N-Cyclohexylanilin, Morpholin, 3,5-Dimethylmorpholin, Piperidin, Pyrrolidin, Thiomorpholin, N-Methylpiperazin, N-Ethylpiperazin, Tetrahydroisochinolin sowie die Methyl- und Ethylester folgender Aminosäuren: Alanin, Arginin, Asparagin, Asparaginsäure, Cystein, Glutamin, Glutaminsäure, Glycin, Histidin, Isoleucin, Leucin, Lysin, Methionin, Phenylalanin, Prolin, Serin, Theorin, Tryptophan, Tyrosin, Valin, t-Butylglycin, Ornithin, Norleucin und Sarcosin und Pipecolinsäure und deren Ethylester, Nipecotinsäure und deren Ethylester, Isonipecotinsäure und deren Ethylester, 4-Piperidon und 4-Piperidon-3-Struktur, wie sie in EP-A 451 407 beschrieben sind, oder TAN-Amine siehe EP-A 511 948.

Als Kohlendioxid kann in das erfindungsgemäße Verfahren das übliche Handelsprodukt, gegebenenfalls auch sogenanntes Trockeneis, eingesetzt werden. Außerdem kann, insbesondere für spezielle Zwecke, z.B. für analytische oder diagnostische Verwendungen oder für solche Verwendungen der daraus herstellbaren Produkte, auch mit Isotopen angereichertes, z.B. $^{13}C$ und/oder $^{14}C$ enthaltendes Kohlendioxid eingesetzt werden.

In das erfindungsgemäße Verfahren können die verschiedensten Alkylierungsmittel eingesetzt werden, beispielsweise Verbindungen der Formel (II)

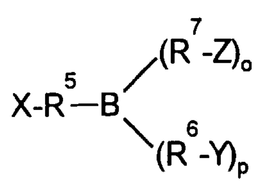

(II),

in der

$R^5$, $R^6$ und $R^7$ gleich oder verschieden sind und jeweils für einen der folgenden Reste in zweibindiger Form stehen: $C_1$-$C_{30}$-Alkyl, $C_4$-$C_{12}$-Cycloalkyl, $C_3$-$C_{30}$-Alkenyl, $C_5$-$C_{12}$-Cycloalkenyl, $C_3$-$C_{30}$-Alkinyl, $C_8$-$C_{12}$-Cycloalkinyl, $C_7$-$C_{20}$-Aralkyl, $C_8$-$C_{20}$-Aralkenyl, $C_8$-$C_{20}$-Aralkinyl, $C_8$-$C_{20}$-Alkenaryl, $C_8$-$C_{20}$-Alkinaryl, $C_5$-$C_{13}$-Alkenhetaryl oder $C_5$-$C_{13}$-Alkinhetaryl, die beiden letzten jeweils mit bis zu 3 Sauerstoff-, Schwefel- und/oder Stickstoffatomen im Ringsystem,

wobei kettenförmige Molekülteile linear, verzweigt und/oder gegebenenfalls durch Sauerstoff-, Schwefel-, N($C_1$-$C_{12}$-Alkyl oder $C_6$-$C_{10}$-Aryl)-, CO-, COO-, SO-, $SO_2$-oder SO(O)O- oder OP($C_1$-$C_{12}$-Alkyl oder $C_6$-$C_{10}$-Aryl)-Gruppen unterbrochen sein können,

wobei die Gruppierungen $R^6$-Y und $R^7$-Z auch für Wasserstoff stehen können und in diesem Falle $R^5$ gegebenenfalls zusätzlich durch COO($C_1$-$C_{12}$-Alkyl oder $C_6$-$C_{10}$-Aryl), CON($C_1$-$C_{12}$-Alkyl oder $C_6$-$C_{10}$-Aryl)$_2$, $C_6$-$C_{10}$-Aryl, O-C(O)-($C_1$-$C_{12}$-Alkyl oder $C_6$-$C_{10}$-Aryl), $CH_2$-O-(-C(O)-($C_1$-$C_{12}$-Alkyl oder $C_6$-$C_{10}$-Aryl), $C_5$-$C_{11}$-Hetaryl mit bis zu 3 Sauerstoff-, Schwefel- und/oder Stickstoffatomen im Ringsystem, O($C_1$-$C_{12}$-Alkyl oder $C_6$-$C_{10}$-Aryl), N($C_1$-$C_{12}$-

Alkyl oder $C_6$-$C_{10}$-Aryl$)_2$, OP(O-$C_1$-$C_{12}$-Alkyl oder $C_6$-$C_{10}$-Aryl$)_2$, Si(O-$C_1$-$C_{12}$-Alkyl oder -$C_6$-$C_{10}$-Aryl$)_3$, Si($C_1$-$C_{12}$-Alkyl oder $C_6$-$C_{10}$-Aryl$)_3$ oder Halogen substituiert sein können,

o und p unabhängig voneinander jeweils für Null oder 1 stehen,

B nicht vorhanden ist, wenn o = p = Null ist,

B im Falle p = 1 und o = Null für einen der folgenden Reste in zweibindiger Form steht: $CH_2$, N($C_1$-$C_{12}$-Alkyl oder $C_6$-$C_{10}$-Aryl), OP($C_1$-$C_{12}$-Alkyl oder $C_6$-$C_{10}$-Aryl), Phenyl, Naphthyl, $(CH_2)_q$ mit q = 2 bis 30 oder $(CH_2)_r$-M-$(CH_2)_s$ mit r und s gleich oder verschieden und jeweils = 1 bis 20 und M = einem Sauerstoff- oder Schwefelatom oder einer $SO_2$- oder N($C_1$-$C_{12}$-Alkyl oder $C_6$-$C_{10}$-Aryl)-Gruppe,

B im Falle p = 1 und o = 1 für einen der folgenden Reste in dreibindiger Form steht: CH, N, (O)P, Phenyl oder Naphthyl und

X, Y und Z unabhängig voneinander für je eine Abgangsgruppe stehen.

Alkene und Alkine können jeweils einfach oder mehrfach ungesättigt sein.

Geeignete Abgangsgruppen X, Y und Z sind z.B. Halogen wie Fluor, Chlor, Brom und Iod, Sulfonat wie Aryl- und Perfluoralkylsulfonat, monosubstituiertes Diazo und monosubstituiertes Nitrato, sowie die zusätzlich in J. March, Advanced Organic Chemistry, 3rd ed., John Wiley & Sons, New York 1985, S. 310-316 aufgeführten. Geeignet sind auch chlormethylierte aromatische Polymere, z.B. Chlormethylpolystyrol, mit Molekulargewichten von z.B. 1000 bis 1.000.000.

Bevorzugte Alkylierungsmittel der Formel (II) sind solche, bei denen $R^5$, $R^6$ und $R^7$ gleich oder verschieden sind und jeweils für einen der folgenden Reste in zweibindiger Form stehen: $C_1$-$C_{20}$-Alkyl, $C_5$-$C_8$-Cycloalkyl, $C_3$-$C_{10}$-Alkenyl, $C_5$-$C_8$-Cycloalkenyl, $C_3$-$C_{10}$-Alkinyl, $C_7$-$C_{14}$-Aralkyl, $C_8$-$C_{15}$-Aralkenyl, $C_8$-$C_{15}$-Aralkinyl, $C_8$-$C_{14}$-Alkenaryl, $C_5$-$C_{10}$-Alkenhetaryl oder $C_5$-$C_{10}$-Alkinhetaryl, die beiden letzten jeweils mit bis zu 2 Schwefel- und/oder Stickstoffatomen im Ringsystem,

wobei kettenförmige Molekülteile linear, verzweigt und/oder gegebenenfalls durch Sauerstoff-, Schwefel-, N($C_1$-$C_{12}$-Alkyl oder $C_6$-$C_{10}$-Aryl)-, CO-, SO- oder $SO_2$-Gruppen unterbrochen sein können,

wobei die Gruppierungen $R^6$-Y und $R^7$-Z auch für Wasserstoff stehen können und in diesem Falle $R^5$ gegebenenfalls zusätzlich durch COO($C_1$-$C_6$-Alkyl oder Phenyl), CON($C_1$-$C_6$-Alkyl), $C_6$-$C_{10}$-Aryl, $C_5$-$C_8$-Hetaryl mit bis zu 2 Schwefel- und/oder Stickstoffatomen im Ringsystem, O-$C_1$-$C_6$-Alkyl, O-C(O)-$C_1$-$C_{12}$-Alkyl, $CH_2$O-(-C(O)-$C_1$-$C_{12}$-Alkyl, N-$C_1$-$C_6$-Alkyl oder Halogen substituiert sein können,

B nicht vorhanden ist, wenn o = p = Null ist,

B im Falle p =1 und o = Null für einen der folgenden Reste in zweibindiger Form steht: $CH_2$, N($C_1$-$C_6$-Alkyl oder Phenyl), Phenyl, Naphthyl, $(CH_2)_q$ mit q = 2 bis 10 oder $(CH_2)_r$-M-$(CH_2)_s$ mit r und s gleich oder verschieden und jeweils = 1 bis 10 und M = einem Sauerstoff- oder Schwefelatom oder einer $SO_2$- oder N($C_1$-$C_6$-Alkyl oder Phenyl)-Gruppe,

B im Falle p = 1 und o = 1 eine oben angegebene Bedeutung hat und

X, Y und Z unabhängig voneinander je für eine Abgangsgruppe stehen.

Als Alkylierungsmittel kommen auch Tri($C_1$-$C_{12}$-alkyl)-phosphite sowie Acetate und Aminalester des Dimethylformamids mit jeweils bis zu insgesamt 10 C-Atomen in Frage, sowie α-D-Glykopyranosyl -bromid-tetraacetat, α-D-Glykopyranosyl-bromid-tetrabenzoat, 2,3,4-Tri-O-acetyl-α-D-Xylopyranosylbromid, 2,3,4,6-Tetra-O-acetyl-α-D-glykopyranosylbromid, Methyl-2,3,4-tri-O-acetyl-1-brom-1-deoxy-α-D-glykopyranuronate, α-D-Galaktopyranosylbromidtetraacetat, Acetobrom-α-D-galaktose, 2,3,4,6-Tetra-O-acetyl-α-D-galaktopyranosylbromid, Methyl-(2,3,4-tri-O-acetyl-α-D-glykopyranosylbromid)-uronat, 1-α-Bromgluconsäure-2,3,4-tri-O-acetylmethylester, 1-α-Bromgluconsäure-2,3,4-tri-O-acetylethylester, Acetobromcellobiose, α-Bromo-hepta-O-acetylmaltose, 2,3,6-Tri-O-acetyl-4-O-(2,3,4,6-tetra-O-acetyl-α-D-glucopyranosyl)-glucosylbromid und ähnliche Halogen-Sauerstoff-Acetale, wie sie beispielsweise beschrieben sind in Houben-Weyl, Methoden der Organischen Chemie, Band E 14a/3, Seiten 1 bis 136 und 621 bis 1075 und Halogen-Schwefel- und Halogen-Stickstoff-Acetale, wie beispielsweise dort beschrieben auf

den Seiten 142 bis 620.

Außer den bisher beschriebenen Reagenzien für das Verfahren der vorliegenden Erfindung kann man auch C(3)-Chlor(oder Brom oder Jod)-1,3-dihydro-2H-1,4-benzodiazepin-2-on herstellen (siehe J. Heterocylic chem. 16 1449 (1979), Methoden A und B) und diese mit Kohlendioxid und Aminen umsetzen und so beispielsweise Camazepam erhalten (= Dimethylcarbaminsäure-7-chlor-2,3-dihydro-1-methyl-2-oxo-5-phenyl-1H-1,4-benzodiazepin-3-yl-ester (siehe Merck Index, 11. Auflage, S. 1732).

Besonders bevorzugte Alkylierungsmittel sind solche der Formel (III)

$$X - R^{5'} \qquad (III),$$

in der

$R^{5'}$ für einen der folgenden Reste in einbindiger Form stehen: $C_1$-$C_{20}$-Alkyl, $C_4$-$C_{12}$-Cycloalkyl, $C_3$-$C_{30}$-Alkinyl, $C_7$-$C_{20}$-Aralkyl, $C_8$-$C_{20}$-Aralkenyl, $C_8$-$C_{20}$-Alkenaryl, $C_8$-$C_{20}$-Alkinaryl, $C_5$-$C_{13}$-Alkenhetaryl oder $C_5$-$C_{13}$-Alkinhetaryl, die beiden letzten jeweils mit bis zu 2 Sauerstoff-, Schwefel- und/oder Stickstoffatomen im Ringsystem und X für eine Abgangsgruppe steht, sowie folgende Einzelverbindungen:

Die Bromide, Jodide, Toluolsulfonate und Mesylate von Methyl-, Ethyl-, Propyl-, i-Propyl-, Allyl-, n-Butyl-, i-Butyl-, s-Butyl-, t-Butyl-, Pentyl-, Hexyl-, Octyl-, Nonyl-, Decyl-, Undecyl-, Dodecyl-, Tridecyl-, Tetradecyl-, Pentadecyl-, Hexadecyl-, Heptadecyl-, Octadecyl-, Nonadecyl-, Eicosyl-, Docosyl- und Tricosyl-Radikalen, Cyclopropylmethylchlorid, Cyclopropylmethylmesylat, Benzylchlorid, Benzylbromid, Benzyliodid, Benzyl-p-tolylsulfonat, Benzylmesylat, 2-(Chlormethyl)-naphthalin, 3-(Chlormethyl)-naphthalin, 9-Fluorenylmethylchlorid, - toluolsulfonat und -mesitylat, Cinnamylchlorid, p-Methoxybenzylchlorid, m- und p-Nitrobenzylchlorid, p-Brombenzylchlorid, o-, m- und p-Chlorbenzylchlorid, 9-Anthrylmethylchlorid, Methylchlormethylether, Ethylchlormethylether, Butylchlormethylether, Octylchlormethylether, N-Chlormethylphthalimid, 2,2,2-Trichlor-1,1,1-dimethylethylchlorid, o-, m- und p-Chlormethylbenzylchlorid, Monochloraceton, Ethylchlormethylketon, Propylchlormethylketon, Phenylchlormethylketon, p-Nitrophenylchlormethylketon, Chloressigsäure-methylester und -ethylester, Bromessigsäure-methylester und -ethylester, p-(Chlormethyl)-polystyrol, Merrifield's Peptid-Harze, 4-Chloracetessigsäure-t-butylester, 2,3-, 2,4-, 2,5-, 2,6- und 3,4-Dichlorbenzylchlorid, (3-Chlorpropyl)-4-methylpiperazin, 1,2-Dichlorethan, 1,3-Dichlorpropan, 1,4-Dichlorbutan, 1,5-Dichlorpentan, Dichlormethylether, 2,4-Dichlor-2-buten, 1,4-Dichlor-2-butin, Di-(1-chlorethyl)-thioether, Chlormethyl-t-butylketon, 3-(Chlormethyl)-heptan, Chloracetonitril, 4-Chlorbutannitril, Chloracetaldehyd, Epichlorhydrin, Isoamylchlorid, 6-Chlorhexylisocyanat, 2-Isocyanatobenzylchlorid, Triethoxychlormethylsilan, 1-Chlorpropionsäuremethylester und -ethylester, Crotylchlorid, 1-Dimethylamino-2-chlorethan, 1,2-Dichlor-2-propen, 1-Chlor-2-methyl-2-propen, Chloracetamidobenzol, 1-Chloracetamido-4-chlorbenzol, N,N-Diethylchloracetamid, 2-Chlorethyl-p-chlorphenylketon, 2- oder 4-Chloracetessigsäuremethylester, -ethylester, -propylester, -n-butylester und -t-butylester, $\alpha$-2,4-Trichloracetophenon, Chlormethylphenyl- und -tolylsulfon, 3-Chlorpentendion, Diethylchloracetamid, Dimethyl-2-chloracetamid, Ethyl-$\alpha$-chlorphenylacetat und 2-Chlormalonsäuredimethyl- und -diethylester. Auch Methylenchlorid und Methylenbromid sowie Chlor-Brom-Methan sind geeignete Alkylierungsmittel.

Besonders bevorzugte Sulfonatreste X, Y und Z sind p-Toluolsulfonat, p-Brombenzolsulfonat, p-Chlorbenzolsulfonat, p-Nitrobenzolsulfonat, Methansulfonat, Trifluormethansulfonat, Nonafluorbutansulfonat, 2,2,2-Trifluorethansulfonat und Benzalsulfonat.

Sulfonatgruppen enthaltende Alkylierungsmittel können, wie andere genannte Alkylierungsmittel auch, separat hergestellt und in das erfindungsgemäße Verfahren als solche eingesetzt werden. Man kann die benötigten Alkylierungsmittel auch in-situ herstellen, Sulfonatogruppen-enthaltende Alkylierungsmittel beispielsweise aus Alkoholen und Halogensulfonverbindungen.

Pro eingesetztes Aminäquivalent kann man in das erfindungsgemäße Verfahren beispielsweise 0,01 bis 10.000 Äquivalente Kohlendioxid einsetzen. Vorzugsweise beträgt dieses Verhältnis 1:0,5 bis 1.000, insbesondere 1:1 bis 10.

Pro eingesetztes Aminäquivalent kann man in das erfindungsgemäße Verfahren beispielsweise 0,01 bis 10.000 Äquivalente Alkylierungsmittel einsetzen. Vorzugsweise beträgt dieses Verhältnis 1:0,3 bis 100, insbesondere 1:0,4 bis 10. Insbesondere bei weniger reaktiven Alkylierungsmitteln kann das Alkylierungsmittel in deutlichem Überschuß eingesetzt werden und dann auch gegebenenfalls als Lösungsmittel dienen.

Es ist ein wesentliches Merkmal der vorliegenden Erfindung, daß man in Gegenwart einer oder mehrerer basischen Verbindungen der Elemente Lithium, Natrium, Magnesium, Kalium, Kalzium, Rubidium, Strontium, Cäsium, Barium und/oder des Ammoniums arbeitet. Als basische Verbindungen kommen z.B. basisch reagierende Salze, Oxide, Hydride und Hydroxide in Frage. Beispielsweise seien genannt: Lithiumhydrid, Natriumhydrid, Kaliumhydrid, Kalziumhydrid, Lithiumhydroxid, Natriumhydroxid, Kaliumhydroxid, Rubidiumhydroxid, Magnesiumhydroxid, Kalziumhydroxid, Strontiumhydroxid, Bariumhydroxid, Lithiumoxid, Natriumperoxid, Kaliumoxid, Kaliumperoxid, Kalziumoxid, Bariumoxid, Magnesiumoxid, Strontiumoxid, Lithiumcarbonat, Lithiumhydrogencarbonat, Natriumcarbonat, Natriumhydrogencarbonat, Kaliumcarbonat, Kaliumhydrogencarbonat, Rubidiumcarbonat, Rubidiumhydrogencarbonat, Cäsiumhydrogencarbonat, Cäsiumcarbonat, Lithiumcyanid, Natriumcyanid, Kaliumcyanid, Rubidiumcyanid, Ammoniumhydrogencarbonat, Ammoniumcarbonat, Ammoniumcarbamat, Kaliumsulfit, Kaliumhydrogensulfid, Natriumsulfid, Natriumhydrogensulfid und/oder deren natürlich vorkommende oder synthetisch erhältliche Gemische, wie beispielsweise Dolomit oder Magnesiumoxidcarbonat und/oder Verbindungen, die Natrium- oder Kaliummetall auf den entsprechenden Carbonaten in dispergierter Form enthalten.

Bevorzugt sind Alkalicarbonate und/oder -hydrogencarbonate, ganz besonders bevorzugt Kaliumcarbonat.

Die basisch reagierenden Verbindungen können in wasserfreier Form oder, soweit es sich um Salze handelt, die mit Hydratwasser kristallisieren, auch in hydrierter Form eingesetzt werden. Bevorzugt werden wasserfreie Verbindungen eingesetzt.

Die basisch reagierenden Verbindungen können beispielsweise in Mengen von 0,5 bis 10 Mol pro Mol eingesetztes Amin verwendet werden. Vorzugsweise liegt diese Menge im Bereich 0,8 bis 5 Mol, besonders bevorzugt im Bereich 1 bis 2,5 Mol, jeweils pro Mol eingesetztes Amin.

Man kann das erfindungsgemäße Verfahren gegebenenfalls in Gegenwart von Hilfsbasen durchführen, d.h. in Gegenwart von weiteren Basen, beispielsweise in einer Menge von weniger als 0,5 Mol, bezogen auf die eingesetzte Base.

Als solche Hifsbasen kommen beispielsweise in Frage: Halogenide von Alkalimetallen, Zeolithe, Kaliumacetat, Kaliumformiat, Natriumacetat, Titanalkoholate, Titansäureamide, Amidinbasen oder Guanidinbasen wie 1,5-Diazabicyclo(4.3.0)non-5-en(DBN), 1,8-Diazabicyclo(5.4.0)undec-7-en (DBU), 7-Methyl-1,5,7-triazabicyclo(4.4.0)dec-5-en (MTBD), Cyclohexyl-tetrabutylguanidin, Cyclohexyl-tetramethylguanidin, N,N,N,N-Tetramethyl-1,8-naphthalindiamin, methylpiperidin, N,N-Dimethylaminopyridin, N-Butyl-tetraethylguanidin, N-t-Butyl-N',N'-diethylformamidin, Tetramethylguanidin, Tetraethylguanidin, N-t-Butyl-N',N'-dimethylacetamidin, N-Cyclohexyl-tetraethylguanidin und N-t-Butyl-tetraethylguanidin sowie 1,4-Diazabicyclo(2.2.2)octan (DABCO), tertiäre Amine wie Triethylamin, Trimethylamin, N-Methylmorpholin, Pyridin und Tetramethylethylendiamin, primäre und sekundäre Amine gleicher Struktur wie das zur Umsetzung eingesetzte Amin, Alkyl- und Arylmetallverbindungen wie Butyl-, Methyl-, Phenyl- und Neoprylithium, sowie Grignardreagentien.

Im allgemeinen ist vorteilhaft, das erfindungsgemäße Verfahren in Gegenwart von Lösungsmitteln durchzuführen. Lösungsmittel werden vorteilhafterweise in einer solchen Menge eingesetzt, daß das Reaktionsgemisch während des ganzen Verfahrens gut rührbar bleibt. Als Lösungsmittel kommen beispielsweise in Frage: Kohlenwasserstoffe wie Petrolether, Benzol, Toluol, Chlorbenzol, Dichlorbenzol, Hexan, Cyclohexan, Methancyclohexan, Pentan, Heptan, Octan und technische Kohlenwasserstoffgemische, beispielsweise sogenannte White Spirits mit Komponenten mit Siedepunkten im Bereich von beispielsweise 40 bis 250°C, Ether wie Dimethyl-, Diethyl-, Dipropyl-, Diisopropyl-, Dibutyl-, Methyl-t-Butylether, Tetrahydrofuran, 1,4-Dioxan und Polyether des Ethylenoxids und/oder Propylenoxids, Amine wie Trimethyl-, Triethyl-, Tripropyl-, Tributylamin, N-Methylmorpholin, Pyridin und Tetramethylethylendiamin, Ester wie Methyl-, Ethyl- und Butylacetat, sowie Dimethyl-, Dibutyl- und Ethylencarbonat, Nitroverbindungen wie Nitromethan, Nitroethan, Nitropropan und Nitrobenzol, Nitrile wie Acetonitril, Propionitril und Benzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid, Ketone wie Aceton, Methylbutylketon und Methylethylketon, verflüssigtes Kohlendioxid, Polyether des Ethylenoxids und/oder Propylenoxids und Amide wie Hexamethylenphosphortriamid, N-Methylpyrrolidon, N-Methylcaprolactam, 1,3-Dimethyl-3,4,5,6-tetrahydro-2(1H)-pyrimidin, Octylpyrrolidon, Octylcaprolactam, 1,3-Dimethyl-2-imidazolindion, Dimethylformamid, Dimethylacetamid, Formamid, Diethylformamid, N-Formylpyrolidin, N-Formylmorpholin, N-Formylpiperidin, N,N'-1,4-Diformylpiperazin, Dipropylformamid und Dibutylformamid. Es kommen auch Sulfone in Frage wie Dimethyl-, Diethyl-, Dipropyl-, Dibutyl-, Dipentyl-, Dihexyl-, Methylethyl-, Ethylpropyl-, Ethylisobutyl- und Pentamethylensulfon in Frage sowie 3-Sulfolen.

Man kann das erfindungsgemäße Verfahren auch ohne Lösungsmittel durchführen, insbesondere dann, wenn man stattdessen für Phasen-Transfer-Reaktionen geeignete Hilfsstoffe zusetzt, beispielsweise quartäre Ammoniumsalze, Polyvinylpyrrolidon, Trioctylphosphinoxid und/oder Acetylaceton.

Den Lösungsmitteln können gegebenenfalls Hilfsstoffe zugefügt werden wie Kryptanden, z.B. Kronenether oder Polyether als Komplexbildner.

Selbstverständlich kann man in das erfindungsgemäße Verfahren auch Gemische von Lösungsmitteln einsetzen.

Bevorzugte Lösungsmittel sind Dimethylformamid und Dimethylsulfoxid und Gemische von diesen mit anderen der genannten Lösungsmittel.

Das erfindungsgemäße Verfahren führt man im allgemeinen so durch, daß man zunächst das basisch reagierende Amin und das Kohlendioxid in Gegenwart einer basischen Verbindung der genannten Elemente zusammenbringt und miteinander reagieren läßt. Das Alkylierungsmittel fügt man vorteilhafterweise erst dann zu, wenn die Umsetzung des Amins mit dem Kohlendioxid weitgehend oder vollständig abgelaufen ist. Das Fortschreiten der Reaktion des Amins mit dem Kohlendioxid kann man z.B. an der Wärmetönung erkennen.

Das erfindungsgemäße Verfahren kann man in beiden Stufen beispielsweise bei Temperaturen von -50 bis +180°C

durchführen. Bevorzugt sind Temperaturen im Bereich -30°C bis +150°C, insbesondere solche im Bereich -10°C bis +100°C.

Der Druck ist beim erfindungsgemäßen Verfahren nicht kritisch. Es kann grundsätzlich bei Normaldruck, aber auch bei erhöhtem oder erniedrigtem Druck gearbeitet werden. Vorzugsweise arbeitet man bei Normaldruck oder bei Drukken bis zu 15 bar. Bei höheren Temperaturen ist es vorteilhaft, unter erhöhtem Druck zu arbeiten, gegebenenfalls auch über 15 bar.

Man kann das erfindungsgemäße Verfahren unter einer Kohlendioxid-Atmosphäre durchführen, aber auch in einer Atmosphäre, die Kohlendioxid und noch andere, vorzugsweise inerte Gase enthält. Arbeitet man in flüssigem Kohlendioxid, so tut man dies vorzugsweise im geschlossenen Gefäß bei dem sich einstellenden Eigendruck.

Das erfindungsgemäße Verfahren hat eine Reihe überraschender Vorteile. Es gestattet auf technisch einfache, wirtschaftlich vorteilhafte Weise die Herstellung von Carbamaten unter Vermeidung der Verwendung von Phosgen, Chlor, Ameisensäureester, Isocyanaten, Carbamoylchloriden und Carbonaten, die in vielen Fällen ganz erheblichen Aufwand für die Anlagensicherheit erfordern. Man kann danach N,N-dialkylierte, O-hochreaktive Alkene und Alkine herstellen, welche auf andere Weise gar nicht oder nur unter großen Schwierigkeiten zugänglich sind, wie $Bu_2N-CO-OCH_2-C{\equiv}C-H$ (siehe Beispiel 13). Man kann es bei milden Reaktionsbedingungen durchführen und die Reaktionsmischungen oft direkt weiterverwenden, beispielsweise für Hydrierungen, Chlorierungen, Bromierungen, Oxidationen, Kondensationen und/oder Polymerisationen.

Es können damit auf einfache Weise radioaktive oder markierte C-Atome in organische Moleküle eingeführt werden. Wenn das hergestellte Carbonat in reiner Form isoliert werden soll, kann dies ebenfalls auf einfache Weise geschehen, beispielsweise, indem man das im Reaktionsgemisch vorliegende Salzgemisch abtrennt, die leicht flüchtigen Bestandteile des Reaktionsgemisches abzieht und dann das Carbamat, beispielsweise durch Destillation oder Kristallisation isoliert. Wenn das Reaktionsgemisch nicht abreagiertes Amin enthält, so kann dieses wie oben beschrieben oder durch Wäsche mit verdünnter Säure entfernt werden, ohne daß das gebildete Carbamat angegriffen wird. Das erfindungsgemäße Verfahren eignet sich, da es heterogen arbeitet, auch für die kontinuierliche Durchführung, wobei dann Lösungsmittel und Base, gegebenenfalls nach Regenerierung, in das Reaktionsgemisch zurückgeführt werden können. Dies ist für das Lösungsmittel besonders günstig, da es ohne Einschaltung einer wässrigen Phase bei der Aufarbeitung die direkte Wiederverwendung erlaubt.

Carbamate sind wichtige Substanzen in der chemischen Industrie, die beispielsweise als Zwischenprodukte für Wirkstoffe oder als Wirkstoffe im Pflanzenschutz (dort insbesondere Insektizide, Pestizide, Fungizide und Herbizide) und im pharmazeutischen Bereich verwendbar sind. Sie können auch als Zwischenprodukte für Kunststoffe, Lacke und Polymere dienen. Durch thermische Eliminierung kann man aus Carbamaten Isocyanate gewinnen. Man kann das erfindungsgemäße Verfahren auch anwenden, um unerwünschte halogenhaltige organische Stoffe aus Abgasen und flüssigen und festen Rückständen zu entfernen, sowie um halogenenthaltende organische Kampfstoffe (wie sie z.B. in Klimmek et al., Chemische Gifte und Kampfstoffe, Hippokrates Verlag Stuttgart 1983, S. 27 ff genannt sind) unschädlich zu machen. Beispiele hierfür sind die Entfernung von Methylchlorid und Chlordimethylether aus Abgasen oder die Beseitigung von Bromaceton, Brombutenonen, Chloracetophenonen, Lost und Stickstofflost.

Das erfindungsgemäße Verfahren kann auch angewendet werden, um primäre oder sekundäre Amine für weitere Reaktionen durch Überführung in Carbamate zu schützen und hinterher daraus wieder die primären oder sekundären Amine freizusetzen. Diese Schutzgruppentechnik ist besonders wichtig auf dem Gebiet der Chemie der Proteine (siehe z.B. T.W. Greene, Protective Groups in Org. Synthesis, Wiley-Interscience, New York 1981, S. 223-249). Einige besonders wichtige Carbamate, die auf erfindungsgemäße Weise vorteilhaft hergestellt werden können, sind folgende: 3-Methyl-2-oxalidon, N-Phenyl-carbaminsäure, Ethyl-N-methyl-N-phenylcarbamat, Ethyl-N-ethyl-N-phenylcarbamat, N-Phenylcarbaminsäureisopropylester, Carbondazim, Isopropyl-3-chlorcarbanilat, 4-Nitrophenyl-methylurethan, 1,3-Propandiol-2-methylen-bis(methylcarbamat), 2-[(Methoxycarbonyl)-methyl-amino]-benzolsäure-(N-methylisatat, 7-Hydroxy-1-naphthalincarbamatsäuremethylester, Ethyldiphenylcarbamat, 3-Ethoxycarbonylaminophenyl-N-phenylcarbamat, Methyl-N-[3-[N-(3-methylphenyl)-carbonyl]-phenyl]-carbamat, $CH_3-N(-CH_2CH_2-O-CO-MH-C_6H_5)_2$, N-L-α-Aspartyl-L-phenylalanin-1-methylester-N-benzylcarbamat, Aspartylbenzylester-N-benzylcarbamat, Aspartylmethylester-N-benzylcarbamat, Aspartylethylester-N-benzylcarbamat, Aspartylsäure-N-benzylcarbamat und die in K.H. Buechel, Pflanzenschutz und Schädlingsbekämpfung, Georg-Thieme-Verlag, Stuttgart, 1977, in den Kapiteln Insektizide, Herbizide, Fungizide, Nematozide, Akarizide und Bakterizide genannten Carbamate, sowie die in EP-A 289 842 und DE-A 4 026 966 beschriebenen..

Ebenfalls herstellbar sind Carbamate des Typs wie in WO 93/7116 und EP-A 582 902 beschrieben (siehe Beispiele 33 und 45 der vorliegenden Beschreibung), wobei dann die entsprechenden Benzylverbindungen mit Abgangsgruppen wie oben beschrieben und die entsprechenden Amine als Edukte Verwendung finden.

Auch Carbamate des Typs wie in EP-A 560 424 beschrieben sind auf die erfindungsgemäße Weise herzustellen, insbesondere die Verbindungen 1 bis 3, 6 bis 10, 12 und 14 bis 17, sowie ähnliche Verbindungen mit carbamatartiger Struktur wie in EP-A 560 424 beschrieben. Die Ausgangsmaterialien sind dann z.B. Benzyl- oder aliphatische Verbindungen mit Abgangsgruppen wie oben beschrieben und die entsprechenden Amine.

Auch Carbamate des Typs wie in EP-A 335 297 oder EP-A 366 189 oder von Keith et al., Spec. Publ. R. Soc. Chem. 119, 79 (1993) beschrieben oder in darin zitierten Literaturstellen beschrieben können auf die erfindungsgemäße Weise hergestellt werden. Besonders trifft dies für Verbindungen zu, bei denen Ester von Ciprofloxazin und ähnlichen Chinolon-Derivaten und Cephalosporine in ihrer geschützten Form mit Abgangsgruppen (siehe oben) eingesetzt werden. Bevorzugte aktivierte Ester sind Tosyl- und Mesylester.

Auch Carbamate des Typs wie in US-PS 5 210 224 beschrieben sind auf die erfindungsgemäße Weise herzustellen. In diesem Fall sind die entsprechenden Mesyl-, Tosyl- oder andersartigen Acylgruppen an Lankazidin gebunden, statt an die Carbonatgruppe, die in der US-PS genannt ist. Die Reaktion mit Kohlendioxid und Amin kann erfolgen wie oben beschrieben. Die Produkte sind die gleichen wie in der US-PS angegebenen und können auch andere sein, je nach dem eingesetzten Amin.

**Beispiele**

Die in den Beispielen verwendeten Basen (häufig Kaliumcarbonat) wurden bei 150°C und 15 mbar 14 Stunden lang getrocknet und gepulvert. Verwendete Lösungsmittel (häufig Dimethylformamid oder Dimethylsulfoxid) wurden mit einem Molekularsieb von 3**Å** getrocknet. Alle Reaktionen wurden so durchgeführt, daß ein ständiger, schwacher Kohlendioxid-Strom über das Reaktionsgemisch strömte.

**Beispiel 1**

N-Methyl-N-9-fluorenyl-cinnamylcarbamat

5,0 g N-Methyl-N-9-fluorenylamin, 20,0 g Kaliumcarbonat und 100 ml Dimethylformamid (im folgendem mit DMF abgekürzt) wurden mit 40 g Trockeneis versetzt und nach 1 Stunde bei 25°C unter Kohlendioxid-Atmosphäre 4,69 g Cinnamylchlorid zugesetzt. Es wurde 1 Stunde bei 25°C und 5 Stunden bei 50°C nachgerührt, danach vom Feststoff abgetrennt, unter vermindertem Druck die flüchtigen Komponenten vertrieben, mit Dichlormethan/Wasser extrahiert und über Kieselgel mit Toluol und steigendem Ethylacetat-Gehalt chromatographiert. Es wurden 4,96 g (54 % der Theorie) des Zielproduktes mit einem Schmelzpunkt von 108 bis 109°C erhalten. Daneben wurden 0,61 g (8 % der Theorie) N-Methyl-N-9-fluorenyl-N-cinnamylamin vom Schmelzpunkt 85 bis 89°C erhalten.

**Beispiel 2**

N-Methyl-N-9-fluorenyl-cinnamylcarbamat-$^{13}C$

Beispiel 1 wurde wiederholt, jedoch wurde statt Trockeneis $^{13}CO_2$ (entwickelt aus Bariumcarbonat$^{13}C$, 88%ig und Schwefelsäure) eingeblasen. Die Ausbeute betrug 3,14 g (34 % der Theorie). Das $^{13}C$-NMR-Spektrum zeigte eine drastische Intensitätserhöhung des Carbamat-$^{13}C$ bei $\delta$ 157-158 ppm.

**Beispiel 3**

N-Methyl-N-9-fluorenyl-(4-tert.-butylbenzyl)-carbamat

5,0 g N-Methyl-N-9-fluorenylamin wurden in 100 ml DMF und 20,0 g Kaliumcarbonat mit 6,99 g 4-tert.-Butylbenzylbromid versetzt, 2 Stunden bei 25°C und 8 Stunden bei 50°C gerührt und wie in Beispiel 1 beschrieben aufgearbeitet. Es wurden 3,4 g (38 % der Theorie) N-Methyl-N-9-fluorenyl-(4-t-butylamin) mit einem Schmelzpunkt von 92 bis 94°C erhalten, sowie 4,02 g eines Öles, das ca. 80 Gew.-% der Zielsubstanz enthielt. Im IR-Spektrum zeigte sich eine charakteristische Bande (CO-Valenzschwingung) bei 1.685 $cm^{-1}$.

**Beispiel 4**

N-Methyl-N-(1-naphthylmethyl)-cinnamylcarbamat

8,5 g N-Methyl-N-(1-naphthylmethyl)-amin (gewonnen aus dem Hydrochlorid durch Schütteln mit Natriumhydroxid, Extraktion und Kugelrohrdestillation), 38,43 g Kaliumcarbonat und 200 ml DMF wurden 1 Stunde bei 25°C mit Kohlendioxid begast, dann 8,34 g Cinnamylchlorid zugegeben, 1 Stunde bei 25°C und 8 Stunden bei 50°C gerührt und dann wie in Beispiel 1 beschrieben aufgearbeitet. Es wurden 7,23 g (44 % der Theorie) der Zielsubstanz erhalten ($\delta$H: 4,83 ppm, 4,97 ppm). Außerdem wurden 0,83 g (6 % der Theorie) N-Methyl-N-(1-naphthylmethyl)-cinnamylamin isoliert.

**Beispiel 5**

N-Methyl-N-Phenylcinnamylcarbamat

5,35 g N-Methylanilin, 38,43 g Kaliumcarbonat, 200 ml DMF, eingeleitetes Kohlendioxid und 8,34 g Cinnamylchlorid wurden wie in Beispiel 4 beschrieben zur Reaktion gebracht und wie in Beispiel 1 beschrieben aufgearbeitet. Die Ausbeute am Zielprodukt betrug 1,61 g (12 % der Theorie). Die $^{1}H$-NMR- und $^{13}C$-NMR-Spektren zeigten folgende charakteristischen Signale: δH: 4,76 und 3,32 ppm und δC: 155,4 ppm.

**Beispiel 6**

N,N-Dibutyl-cinnamylcarbamat

20,0 g Dibutylamin, 37,73 g Kaliumcarbonat und 100 ml DMF wurden mit Kohlendioxid begast (exotherme Reaktion bis zu ca. 45°C). Danach wurden bei 10°C 23,64 g Cinnamylchlorid zugetropft, 1 Stunde bei 25°C und 4 Stunden bei 50°C gerührt. Nach der Aufarbeitung wie in Beispiel 1 beschrieben, wurden 9,86 g des Zielproduktes und 0,56 g (2 % der Theorie) Dibutylcinnamylamin isoliert. Charakteristische Signale in $^{1}H$-NMR-Spektrum lagen bei 4,73 und 3,23 ppm.

**Beispiel 7**

N,N-Dibutyl-phenylacyl-carbamat

20,0 g Dibutylamin, 37,73 g Kaliumcarbonat und 100 ml DMF wurden wie in Beispiel 6 beschrieben zusammengegeben und bei 25°C 1 Stunde mit Kohlendioxid begast, dann bei 10°C mit 23,95 g α-Chloracetophenon versetzt, dann 1 Stunde bei 25°C und 4 Stunden bei 50°C nachgerührt. Die Aufarbeitung erfolgte durch Extraktion mit 5 gew.-%igem wäßrigem Chlorwasserstoff und Ethylacetat und Destillation im Kugelrohr. Mit einem Siedepunkt von 145°C bei 0,1 mbar wurden 29,1 g des Zielproduktes (62 % der Theorie) erhalten. Die charakteristischen Signale im $^{1}H$-NMR-Spektrum lagen bei 5,31 und 3,28 ppm.

**Beispiel 8**

Beispiel 7 wurde wiederholt, jedoch wurde jeweils nur die halbe Menge eingesetzt, statt DMF Dimethylsulfoxid (im folgenden mit DMSO abgekürzt) verwendet und 6 Stunden bei 25°C gerührt. Danach ergab die gaschromatographische Untersuchung des Reaktionsgemisches eine Ausbeute an Zielprodukt bezogen auf den Umsatz von 98,4 %.

**Beispiel 9**

N,N-Dibutyl-(3-oxo-2-ketobutyl)-carbamat

Es wurde gearbeitet wie in Beispiel 7, jedoch wurde statt 2-Chloracetophenon 23,73 g Bromessigsäureethylester zugegeben und 1 Stunde bei 25°C und 3 Stunden bei 50°C gerührt. Mit einem Siedepunkt von 100°C bei 0,1 mbar wurden 5,0 g des Zielproduktes (12 % der Theorie) isoliert. Die charakteristischen Signale im $^{1}H$-NMR-Spektrum lagen bei 4,60, 4,21 und 3,25 ppm.

**Beispiel 10**

Es wurde verfahren wie in Beispiel 9, jedoch wurde nur die halbe Menge eingesetzt und DMSO statt DMF verwendet. Die Ausbeute (Gaschromatographie, Flächen-%) nach 4 Stunden Reaktionszeit betrug bezogen auf den Umsatz 52 %. Der Umsatz betrug 90 %.

**Beispiel 11**

N-Butylpropargylcarbamat

30 g Butylamin, 100 g Kaliumcarbonat und 160 g DMF wurden mit Kohlendioxid begast (exotherme Reaktion bis 66°C). Nach 1 Stunde wurden bei 15°C 33,68 g 3-Chlorpropin zugesetzt und 1 Stunde bei 25°C und 3 Stunden bei 50°C gerührt. Danach wurde wie in Beispiel 7 beschrieben aufgearbeitet. Es wurden 2,86 g des Zielprodukts (4 % des

Theorie) erhalten.

**Beispiel 12**

5,66 g Butylamin, 18,87 g Kaliumcarbonat und 50 ml DMSO wurden nach 1 Stunde Begasung mit Kohlendioxid bei 25°C mit 5,77 g Propargylchlorid versetzt und 4 Stunden bei 25°C gerührt. Im übrigen wurde verfahren wie in Beispiel 11 beschrieben. Die Ausbeute an N-Butylpropargylcarbamat betrug 87 % bezogen auf den Umsatz (Gaschromatographie, Flächen-%).

**Beispiel 13**

N,N-Dibutylpropargylcarbamat

100 g Dibutylamin, 189,0 g Kaliumcarbonat und 500 ml DMF wurden in einem 3 l-Edelstahlautoklaven mit 440 g Kohlendioxid versetzt und anschließend 30 Minuten auf 100°C erwärmt. Nach dem Abkühlen auf 50°C wurden bei einem Druck von 75 bar 60,0 g 95%iges Propargylchlorid zugegeben und 6 weitere Stunden bei 50°C gehalten. Nach dem Entspannen wurde abgesaugt, der Filterkuchen mit 1 l Methylenchlorid gewaschen und die organische Phase destilliert. Es wurden zwei das Zielprodukt enthaltende Fraktionen aufgefangen. 1. Fraktion: Siedepunkt 55 bis 95°C bei 1,3 bis 1,5 mbar: 11,41 g (50 Gew.-%ig am Zielprodukt). 2. Fraktion: Siedepunkt 97 bis 98°C bei 1,4 mbar: 107,53 g (98,8 Gew.-%ig am Zielprodukt). Das entspricht einer Ausbeute von 69,2 % der Theorie. Charakteristische Signale im $^1$H-NMR-Spektrum lagen bei 4,68, 3,23 und 2,43 ppm.

**Beispiel 14**

N-Methyl-N-phenyl-butylcarbamat

8,29 g N-Methylanilin, 18,87 g Kaliumcarbonat und 70 ml DMSO wurden nach 1 Stunde Begasung mit Kohlendioxid mit 7,17 g Butylchlorid versetzt. Nach 15 Stunden bei 25°C wurde gaschromatographisch die Bildung des Zielprodukts in einer Ausbeute von 42,1 % ermittelt. Das Massenspektrum zeigte $M^+$ bei 207.

**Beispiel 15**

N,N-Dibutyl-benzylcarbamat

10 g Dibutylamin, 18,87 g Kaliumcarbonat und 50 ml DMSO wurden 1 Stunde mit Kohlendioxid begast (leicht exotherme Reaktion). Nach Zugabe von 9,83 g Benzylchlorid wurde 29 Stunden bei 25°C gerührt. Gemäß gaschromatographischer und massenspektroskopischer Analyse lag dann das Zielprodukt in 95,5%iger Ausbeute vor. $M^+$ = 263. Nach Aufarbeitung wie in Beispiel 1 wurden 18,4 g Produkt (90 % der Theorie, 96 % rein) erhalten.

**Beispiel 16**

N,N-Dibutyl-O-[4-(3-methoxy)-buten-2-säure-methylester]-carbamat

10 g Dibutylamin, 18,87 g Kaliumcarbonat und 50 ml DMSO wurden mit Kohlendioxid 1 Stunde begast (leicht exotherme Reaktion) und dann 12,75 g 4-Chlor-3-methoxy-2-butensäuremethylester zugesetzt. Nach 45 Stunden Rühren bei 25°C lag das Zielprodukt gemäß gaschromatographischer und massenspektroskopischer Untersuchung im Reaktionsgemisch in einer Ausbeute von 92,4 % vor. Die Aufarbeitung durch Absaugen und Einengen des Rückstands am Rotationsverdampfer, Aufnehmen mit Ethylacetat und Waschen mit 5 %igem wäßrigem Chlorwasserstoff ergab 26,75 g 79,4 %iges Zielprodukt (90,9 % der Theorie), Rest = Dimethylsulfoxid. Das $^1$H-NMR-Spektrum zeigt die charakteristischen Signale bei 5,23, 5,10 und 3,20 ppm.

**Beispiel 17**

cis/trans - N,N-Dibutyl-O-(crotonsäuremethylester)-carbamat

10,0 g Dibutylamin, 18,87 g Kaliumcarbonat und 50 ml DMSO wurden 1 Stunde mit Kohlendioxid begast (exotherme Reaktion). Dann wurden 16,32 g 4-Bromcrotonsäuremethylester (85%ig) zugesetzt. Nach 45 Stunden Rühren bei 25°C wurde gemäß gaschromatographischer und massenspektroskopischer Analyse das Zielprodukt mit einer Ausbeute von 23,8 % der Theorie gefunden. $M^+ = 271$, cistrans-Gemisch ca. 1:2.

**Beispiel 18**

Tributylcarbamat

10,0 g Dibutylamin, 18,87 g Kaliumcarbonat und 50 ml DMSO wurden bei 25°C mit Kohlendioxid 1 Stunde begast (exotherme Reaktion). Nach Zugabe von 10,62 g Butylbromid wurde nach 45-stündigem Rühren bei 25°C das Zielprodukt gemäß gaschromatographischer und massenspektroskopischer Analyse in einer Menge von 76,0 % der Theorie gefunden. $M^+ = 229$.

**Beispiel 19**

N-Butyl-benzylcarbamat

5,66 g Butylamin, 18,87 g Kaliumcarbonat und 50 ml DMSO wurden 1 Stunde bei 25°C mit Kohlendioxid begast. Nach Zugabe von 9,81 g Benzylchlorid wurde nach 45-stündigem Rühren bei 25°C nach einem Umsatz von 78 % gaschromatographisch und massenspektrometisch das Zielprodukt in einer Ausbeute von 87,2 % der Theorie ermittelt. $M^+ = 207$. Nach Aufarbeitung wie in Beispiel 16 beschrieben, wurden 14,7 g (73 %, 80 %ig) des Zielmoleküls erhalten. $\delta H$: 5,09 und 3,17 ppm.

**Beispiel 20**

N-Methyl-N-phenyl-benzylcarbamat

8,29 g N-Methylanilin, 18,87 g Kaliumcarbonat und 50 ml DMSO wurden 1 Stunde bei 25°C mit Kohlendioxid begast. Dann wurden 9,81 g Benzylchlorid zugesetzt und nach 45-stündigem Rühren bei 25°C gemäß gaschromatographischer und massenspektroskopischer Untersuchung das Vorliegen des Zielprodukts in einer Ausbeute von 56,2 % der Theorie ermittelt ($M^+ = 241$). Daneben hat es sich in einer Ausbeute von 37,0 % der Theorie N-Methyl-N-phenylbenzylamin gebildet. Nach Aufarbeitung wie in Beispiel 1 beschrieben, wurden 7,8 g (42 %) des Zielprodukts isoliert. $\delta H$: 5,15 und 3,30 ppm.

**Beispiel 21**

N-Methyl-N-phenyl-propagylcarbamat

8,29 g N-Methylanilin, 18,87 g Kaliumcarbonat und 50 ml DMSO wurden 1 Stunde bei 25°C mit Kohlendioxid begast. Anschließend wurden 5,78 g Propagylchlorid zugesetzt und 45 Stunden bei 25°C nachgerührt. Nach einem Umsatz von 76 % wurde gemäß gaschromatographischer und massenspektroskopischer Untersuchung das Vorliegen des Zielprodukts in einer Ausbeute von 74,4 % der Theorie ermittelt ($M^+ = 189$). Daneben hatte sich in einer Ausbeute von 20,3 % der Theorie N-Methyl-N-phenyl-N-propagylamin gebildet. Nach Aufarbeitung wie in Beispiel 16 beschrieben, wurden 7,7 g (52 %, 80 %ig) des Zielprodukts isoliert. IR: 1705 $cm^{-1}$, $\delta H$: 4,71 ppm.

**Beispiel 22**

N-Methyl-N-phenyl-allylcarbamat

8,29 g N-Methylanilin, 18,87 g Kaliumcarbonat und 50 ml DMSO wurden 1 Stunde mit Kohlendioxid begast und dann 5,93 g Allylchlorid zugesetzt. Nach 45 Stunden bei 25°C wurden nach einem Umsatz von 31 % das Vorliegen des Zielprodukts in einer Ausbeute von 64,5 % der Theorie gaschromatographisch und massenspektrometisch ermittelt ($M^+ = 191$). Daneben hatte sich eine Ausbeute von 30,3 % der Theorie N-Methyl-N-phenyl-N-allylamin gebildet.

**Beispiel 23**

N-Methyl-N-phenyl-O-[4-(3-methoxy)-buten-2-säure-methylester]-carbamat

8,29 g N-Methylanilin, 18,87 g Kaliumcarbonat und 50 ml DMSO wurden 1 Stunde bei 25°C mit Kohlendioxid begast. Danach wurden 12,79 g 4-Chlor-3-methoxy-2-butensäuremethylester zugefügt und 45 Stunden bei 25°C gerührt. Danach wurde der Ansatz gaschromatographisch und massenspektrometisch untersucht. Das Zielprodukt lag bei einer Ausbeute von 63,4 % der Theorie vor ($M^+$ = 279), daneben in einer Ausbeute von 21,6 % der Theorie N-Methyl-N-phenyl-N-[4-(3-methoxy)-butan-2-säure-methylester]-amin. Nach Aufarbeitung wie in Beispiel 1 beschrieben, wurden 7,1 g (33 %) des Zielprodukts (δH: 5,27, 5,09, 3,67 und 3,31 ppm) mit einem Schmelzpunkt von 49-50°C isoliert, daneben 1,85 g N-Methyl-N-phenyl-4-(3-methoxy)-buten-2-säure-methylester.

**Beispiel 24**

N,N-Dibutylpropagylcarbamat

10,0 g Dibutylamin, 18,87 g Kaliumcarbonat und 250 ml DMSO wurden 1 Stunde bei 25°C mit Kohlendioxid begast. Danach wurde auf einmal 5,78 g Propagylchlorid zugegeben und 4 Stunden nachgerührt. Die gaschromatographische Analyse ergab dann das Vorliegen des Zielprodukts in einer Ausbeute von 99,4 % der Theorie. Daneben hatte sich zu 0,6 % der Theorie N,N-Dibutylpropagylamin gebildet.

**Beispiel 25**

N,N-Dibutylpropagylcarbamat

200 g Dibutylamin, 378,12 g Kaliumcarbonat und 4000 ml DMSO wurden 150 min bei 100 mbar Überdruck mit Kohlendioxid begast (exotherme Reaktion bis ca. 35°C) und dann mit 115,5 g Propagylchlorid (95 %ig) versetzt. Nach 27 Stunden bei 25°C wurde abgesaugt und destilliert. Man erhielt neben den Fraktionen, die bis 68°C bei 8 mbar übergingen und überwiegend DMSO enthielten (zusammen mit 57 g eines Carbamats), 215 g des Zielproduktes in 99 %iger Reinheit mit einem Siedepunkt von 85-95°C bei 0,6 bis 0,9 mbar. Das entspricht insgesamt einer Ausbeute von 272 g (88 % der Theorie).

**Beispiel 26**

N,N-Dibutylallylcarbamat

500 g Dibutylamin, 94,55 g Kaliumcarbonat und 250 ml DMSO wurden 1 Stunde lang mit Kohlendioxid begast und dann mit 29,95 g Allylchlorid versetzt. Die Mischung war sehr viskos und wurde deshalb 30 Tage lang gerührt. Dabei wurde ständig Kohlendioxid eingeleitet und nach 20 und 23 Tagen je 14,8 g Allylchlorid zugesetzt. Nach Absaugen und Destillation wurden 24,95 g N,N-Dibutylallylcarbamat erhalten (30,2 % der Theorie). Das Produkt hatte einen Siedepunkt von 69°C bei 0,1 mbar.

**Beispiel 27**

Sarcosinpropagylcarbamat

11,9 g Sarcosinethylesterhydrochlorid, 29,55 g Kaliumcarbonat und 50 ml DMSO wurden 1 Stunde mit Kohlendioxid begast und dann mit 5,78 g Propagylchlorid versetzt. Nach 22 Tagen wurde abgesaugt, mit Methylenchlorid verdünnt, mit 5 %iger wäßriger Salzsäure gewaschen und die organische Phase eingeengt. Es wurden 6,3 g eines dunklen Öls erhalten, das entspricht 37 % der Theorie, und das Produkt war 91 % rein. $M^+$ = 199, Basis 126. δH: 4,72 und 4,0 ppm.

**Beispiel 28**

Sarcosinpropargylcarbamat

8,0 g Sarcosinethylesterhydrochlorid wurden mit 20 %iger wäßriger Natronlauge in Diethylether bei 0°C behandelt. Die organische Phase getrocknet und eingeengt. Dann wurden 12,81 g Kaliumcarbonat und 50 ml DMSO hinzugefügt. Nach 1 Stunde Begasung mit Kohlendioxid wurden 3,88 g Propagylchlorid zugesetzt. Nach 30 Stunden bei 25°C wurde eine gaschromatographische Analyse durchgeführt, die das Vorliegen von 84,4 % des Zielproduktes ergab.

**Beispiel 29**

4-Chlor-2-butyryl-3-chlorcarbanil

9,89 g 3-Chloranilin, 18,87 g Kaliumcarbonat und 50 ml DMSO wurden 1 Stunde bei 25°C mit Kohlendioxid begast und dann 9,54 g 1,4-Dichlorbut-2-in zugesetzt. Nach 43 Stunden wurden in 12 %iger Ausbeute gaschromatisch und massenspektroskopisch das Zielprodukt analysiert. Das Massenspektrum war identisch mit dem des bekannten Herbizids Barban, bei dem es sich ebenfalls um 4-Chlor-2-butyryl-3-chlorcarbanil handelt.

**Beispiel 30**

Polymeres mit Einheiten der Formel

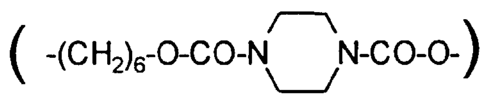

71 g Piperazin, 450 g Kohlendioxid und 500 ml DMSO wurden 30 min bei 100°C gehalten und dann bei 50°C 200 g 1,6-Dibromhexan zugegeben und 10 Stunden bei 70°C gehalten. Danach wurde entspannt, das Gemisch am Rotationsverdampfer eingeengt und schließlich mit 3 l Methylenchlorid extrahiert. Die organische Phase wurde erneut eingeengt, wobei ein wasserlösliches Polymer mit folgenden Kennzahlen erhalten wurde:

$M_w$ = 1057 g/mol, u = 0,68
$M_n$ = 630 g/mol
$M_z$ = 1920 g/mol, $u_z$ = 0,82.

Die Ermittlung der Kennzahlen erfolgte nach der GPC-Methode.

**Beispiel 31**

Polymeres mit Einheiten der Formel

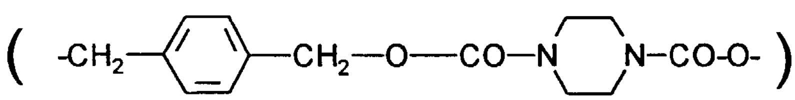

49,1 g Piperazin, 423 g Kaliumcarbonat, 1250 ml DMSO und 200 g Kohlendioxid wurden in einem Edelstahl-Autoklaven 30 min auf 100°C erhitzt und dann 100 g α,α-Dichlor-p-xylol zugesetzt. Nach 6 Stunden bei 50°C und weiteren 6 Stunden bei 80°C wurde nach Aufarbeitung wie in Beispiel 30 ein Polymeres erhalten, dessen Kennzahlen wie in Beispiel beschrieben bestimmt wurden.

$M_w$ = 2443 g/mol, u = 1,49
$M_n$ = 982 g/mol
$M_z$ = 4727 g/mol, $u_z$ = 0,93.

**Beispiel 32**

N-3-Pyridyl-O-4-(3-methoxy-2-butensäuremethylester)carbamat

7,29 g 3-Aminoyridin, 18,87 g Kaliumcarbonat und 50 ml DMSO wurden 1 Stunde lang mit Kohlendioxid begast und dann mit 15,55 g 4-Chlor-3-methoxy-2-butensäuremethylester versetzt. Nach 116 Stunden bei 25°C wurden chromatographisch und massenspektroskopisch 43 % der Theorie des produktes analysiert. $M^+$ = 266, Basis: 121).

**Beispiel 33**

Verbindung identisch mit der des Beispiels 2/2 aus WO 93/7116

1,54 g 3-Trifluormethyl-N-methylanilin, 150 ml DMSO und 4,87 g Kaliumcarbonat wurden 2 Stunden bei 1,1 bar mit Kohlendioxid begast und dann 2,5 g 2-(2-Brommethylphenyl)-3-methoxyacrylsäuremethylester zugesetzt und 6 Tage bei 25°C gerührt. Nach Aufarbeitung wie in Beispiel 1 wurden 2,2 g (59 % der Theorie, 86 %ig) des Zielmoleküls erhalten. Das $^1$H-NMR-Spektrum der hergestellten Verbindung war mit dem aus WO 93/7116 bekannten identisch.

**Beispiel 34**

D-Glucopyranosyl-(dibenzylcarbamat)tetraacetat

Eine Mischung aus 4,80 g Dibenzylamin, 40 ml Dimethylsulfoxid und 6,72 g Kaliumcarbonat wurde 1 Stunde bei 25°C gerührt während ein konstanter Strom von Kohlendioxid durch das Reaktionsgefäß geleitet wurde. Danach wurden 10,0 g $\alpha$-D-Glucopyranosylbromid-tetraacetat (97 % rein) hinzugefügt und die Mischung für weitere 28 Stunden gerührt, danach filtriert und das Lösungsmittel verdampft. Das hinterbleibende Öl wurde chromatographiert, wobei KG 60 Silikagel und Toluol als Lösungsmittel benutzt wurde. Die Hauptfraktion kristallisierte nach der Entfernung des Lösungsmittels in einer Ausbeute von 5,87 g und mit einem Schmelzpunkt von 115 bis 116°C. Das $^{13}$C-NMR-Spektrum zeigte Signale bei 170,6, 170,1, 169,4, 169,2 und 154,3 ppm. Das letzte Signal ist durch die Carbamat-Gruppierung verursacht, die anderen Signale durch die Acetat-Gruppierung.

**Beispiel 35**

Propan-2-on-N,N-dibutylcarbamat

Eine Mischung aus 10,0 g Dibutylamin, 21,39 g Kaliumcarbonat und 200 ml Dimethylsulfoxid wurde für 2 Stunden bei 100 mbar überatomosphärischem Druck mit Kohlendioxid behandelt. Danach wurden 7,17 g Chloraceton hinzugefügt und 24 Stunden bei 25°C nachgerührt. Das gewünschte Produkt wurde in einer Ausbeute von 89 % (gaschromatographische Bestimmung) erhalten. Das Massenspektrum zeigte $M^+$ bei 229.

**Beispiel 36**

3-Aza-3-ethyl-pentan-2-on-N,N-dibutylcarbamat

10,0 g Dibutylamin, 21,39 g Kaliumcarbonat und 200 ml Dimethylsulfoxid wurden vermischt und 2 Stunden lang bei 100 mbar überatmosphärischem Druck mit Kohlendioxidgas behandelt. Danach wurden 11,59 g N-(2-Chloracetyl)-diethylamin hinzugefügt und 27 Stunden bei 25°C gerührt. Das gewünschte Produkt wurde in einer Ausbeute von 99 % (gaschromatographische Bestimmung) erhalten. Das Massenspektrum zeigte $M^+$ bei 286.

**Beispiel 37**

1-(Ethoxycarbonyl)-propan-2-on-N,N-dibutylcarbamat

Butylamin, Kaliumcarbonat und Dimethylsulfoxid wurden wie in Beispiel 36 beschrieben miteinander vermischt und mit Kohlendioxid behandelt. Danach wurden 12,75 g Ethyl-2-chloracetoacetat hinzugefügt und 30 Stunden bei 25°C nachgerührt. Das gewünschte Produkt bildete sich mit einer Ausbeute von 46 % (gaschromatographische Bestimmung). Das Massenspektrum zeigte $M^+$ bei 301.

**Beispiel 38**

1-(Dimethylaminocarbonyl)-propan-2-on-N,N-dibutylcarbamat

Dibutylamin, Kaliumcarbonat und Dimethylsulfoxid wurden wie in Beispiel 36 beschrieben mit Kohlendioxid behandelt. Danach wurden 12,67 g Dimethyl-2-chloracetoamid hinzugefügt und 24 Stunden bei 25°C nachgerührt. Das gewünschte Produkt bildete sich mit einer Ausbeute von 71 % (gaschromatographische Bestimmung). Das Massenspektrum zeigte $M^+$ bei 300.

**Beispiel 39**

1-Acetylpropan-2-on-N,N-dibutylcarbamat

Dibutylamin, Kaliumcarbonat und Dimethylsulfoxid wurden mit Kohlendioxidgas behandelt wie in Beispiel 36 beschrieben. Danach wurden 10,35 g 3-Chloracetylaceton hinzugefügt und 24 Stunden bei 25°C nachgerührt. Das gewünschte Produkt bildete sich mit einer Ausbeute von 47 % (gaschromatographische Bestimmung). Das Massenspektrum zeigte $M^+$ bei 271.

**Beispiel 40**

Propargyl-N-methyl-N-benzylcarbamat

88,0 g N-Methyl-N-benzylamin, 200,73 g Kaliumcarbonat und 750 ml Dimethylsulfoxid wurden gemischt und nach Behandlung mit Kohlendioxidgas für 2 Stunden bei 100 mbar überatmosphärischem Druck 54,18 g Propargylchlorid hinzugefügt und 48 Stunden bei 25°C nachgerührt. Danach wurde das Reaktionsgemisch abfiltriert und destilliert. Bei einem Siedepunkt von 123°C bei 0,9 mbar wurden 90,0 g des gewünschten Produkts erhalten (Reinheit 97 %).

**Beispiel 41**

$[(C_6H_5\text{-}CH_2)_2N\text{-}C(O)\ O]_2CH_2$

50,0 g Dibenzylamin, 175,4 g Kaliumcarbonat und 500 ml Dimethylsulfoxid wurden gemischt und für 1 Stunde bei 70°C in einem Autoklaven bei 30 bar mit Kohlendioxidgas behandelt. Danach wurden 88,2 g Methylenbromid hinzugefügt und 16 Stunden bei 70°C nachgerührt. Dann wurde die Reaktionsmischung filtriert und das Lösungsmittel abgedampft. Der zurückbleibende Feststoff wurde mit Hexan gewaschen und getrocknet. Das gewünschte Produkt wurde mit einem Schmelzpunkt von 86 bis 89°C und in einer Ausbeute von 64,4 % der Theorie erhalten.

**Beispiel 42**

Beispiel 41 wurde wiederholt, jedoch wurden anstelle von Methylenbromid 86,3 g Methylenchlorid und 1,66 g Kaliumjodid eingesetzt. Die Ausbeute des gewünschten Produkts betrug 52 % der Theorie.

**Beispiel 43**

Benzyloxycarbonyl-nipecotinsäureethylester

25 g Ethylnipecotinsäureester, 43,95 g Kaliumcarbonat und 200 ml Dimethylsulfoxid wurden gemischt und für 2 Stunden bei 100 mbar überatmosphärischem Druck mit Kohlendioxidgas behandelt. Danach wurden 20,4 g Benzylchlorid hinzugefügt und 9 Tage bei 25°C gerührt. Das Reaktionsgemisch wurde mit 5%iger wäßriger Salzsäure und Ethylacetat gewaschen und mit Natriumsulfat getrocknet. Nach Entfernung des Lösungsmittels im Vakuum blieb das gewünschte Produkt in Form eines Öls zurück, Ausbeute: 25,0 g. Das $^1H$-NMR-Spektrum zeigte wesentliche Signale bei 7,35 ppm (5H, Phenyl) und 5,13 ppm (2H,s).

**Beispiel 44**

(1-Naphthylmethyl)-N,N-dimethylcarbamat

10 g eines Umsetzungsproduktes aus 2 mol Dimethylamin und 1 mol Kohlendioxid (Handelsprodukt: Dimcarb, Fa. Schuchardt, Deutschland), 41,19 g Kaliumcarbonat und 200 ml Dimethylsulfoxid wurden vermischt und für 2 Stunden bei 100 mbar überatmosphärischem Druck mit Kohlendioxidgas behandelt. Danach wurden 26,30 g (1-Chlormethyl)-naphthalin hinzugefügt und 65 Stunden bei 25°C weitergerührt. Das Reaktionsgemisch wurde aufgearbeitet wie in Beispiel 1 beschrieben. Das gewünschte Produkt wurde in einer Ausbeute von 12,13 g (71 %) erhalten.
$^1H$-NMR: 5,57, 2,93 und 2,86 ppm

**Beispiel 45**

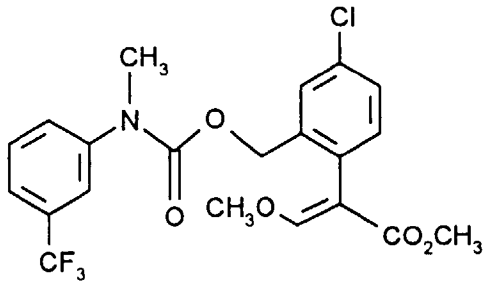

Eine Lösung von 40 g Natriumhydrid (60%ig) in 1000 ml Dimethylformamid wurde mit 100 g Methyl-2-(2-methyl-4-chlorphenyl)-essigsäuremethylester (siehe US-PS 4 424 394) bei 20°C innerhalb von 20 Minuten versetzt. Nach 2-stündigem Rühren bei 2°C wurden 189 g Dimethylsulfat zugetropft und das Gemisch über Nacht stehen gelassen. Danach wurde das Gemisch mit Methylenchlorid extrahiert, mit einem Eis/Wasser-Gemisch versetzt, die organische Phase abgetrennt, über Natriumsulfat getrocknet und destilliert. Der Siedepunkt lag bei 121 bis 135°C bei 0,25 mmHg Druck. Auf diese Weise wurden 104,9 g Methyl-2-(2-methyl-4-chlorphenyl)-3-methoxyacrylat erhalten.

27,37 g dieses Produkts, 350 ml Tetrachlormethan und 22,45 g N-Bromsuccinimid wurden nach Zugabe von 0,5 g Dibenzoylperoxid 90 Minuten am Rückfluß erhitzt. Nach Filtration und Verdampfung des Lösungsmittels wurden 24 g Methyl-2-(2-brommethyl-4-chlorphenyl)-3-methoxyacrylat mit einem Schmelzpunkt von 48°C erhalten.

Diese Verbindung (8,6 g) wurde wie in Beispiel 33 für einen anderen Acrylsäuremethylester beschrieben umgesetzt, wobei 27,08 g Kaliumcarbonat und 8,6 g 3-Trifluormethyl-N-methylanilin zum Einsatz kamen. Die Aufarbeitung des Reaktionsgemisches erfolgte ebenfalls wie in Beispiel 33. Das gewünschte Produkt wurde so in einer Ausbeute von 11,01 g erhalten. Das $^1H$-NMR-Spektrum wies folgende Signale auf: 7,56 ppm, s(2H), 7,47 ppm, s(2H), 7,25 ppm, dd(2H), 7,06 ppm, d(1H), 5,05 ppm, s(2H), 3,79 ppm, s(3H), 3,67 ppm, s(3H) und 3,34 ppm, s(2H).

**Patentansprüche**

1. Verfahren zur Herstellung von organischen Carbamaten aus einem basisch reagierenden Amin, Kohlendioxid und einem Alkylierungsmittel, dadurch gekennzeichnet, daß man zunächst das Amin mit Kohlendioxid in Gegenwart von einer oder mehreren basischen Verbindungen der Elemente Lithium, Natrium, Magnesium, Kalium, Kalzium, Rubidium, Strontium, Cäsium, Barium und/oder des Ammoniums zur Reaktion bringt und dann das Alkylierungsmittel hinzufügt.

2. Verfahren nach Anspruch 1, dadurch gekennzeichnet, daß man als basisch reagierende Amine solche der Formel (I) einsetzt

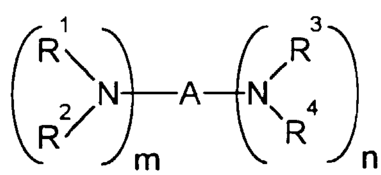

(I),

in der

$R^1$, $R^2$, $R^3$ und $R^4$ gleich oder verschieden sind und jeweils für Wasserstoff oder einen der folgenden Reste in einbindiger Form stehen: $C_1$-$C_{30}$-Alkyl, $C_3$-$C_{30}$-Alkenyl, $C_3$-$C_{30}$-Alkinyl, $C_3$-$C_{12}$-Cycloalkyl, $C_5$-$C_{12}$-Cycloalkenyl, $C_8$-$C_{12}$-Cycloalkinyl, $C_6$-$C_{14}$-Aryl, $C_5$-$C_{13}$-Hetaryl mit bis zu 3 Sauerstoff-, Schwefel- und/oder Stickstoffatomen im Ringsystem, $C_7$-$C_{20}$-Aralkyl, $C_9$-$C_{20}$-Aralkinyl, $C_9$-$C_{20}$-Aralkinyl, $C_7$-$C_{20}$-Alkaryl, $C_8$-$C_{20}$-Alkenaryl oder $C_8$-$C_{20}$-Alkinaryl,

die gegebenenfalls ein- bis fünfmal durch O-$C_1$-$C_{12}$-Alkyl oder -$C_6$-$C_{10}$-Aryl, $NH_2$, NH-$C_1$-$C_{12}$-Alkyl oder -$C_6$-

$C_{10}$-Aryl, N($C_1$-$C_{12}$-Alkyl oder -$C_6$-$C_{10}$-Aryl)$_2$, COO-$C_1$-$C_{12}$-Alkyl oder COO-$C_6$-$C_{10}$-Aryl, $CONH_2$, CONH-$C_1$-$C_{12}$-Alkyl, CON($C_1$-$C_{12}$-Alkyl)$_2$, Halogen, OP(O-$C_1$-$C_{12}$-Alkyl oder -$C_6$-$C_{10}$-Aryl)$_2$, Si($C_1$-$C_{12}$-Alkyl und/oder $C_6$-$C_{10}$-Aryl)$_3$, Si(O-$C_1$-$C_{12}$-Alkyl und/oder $C_6$-$C_{10}$-Aryl)$_3$, Si($C_1$-$C_{12}$-Alkyl oder -$C_6$-$C_{10}$-Aryl)(O-$C_1$-$C_{12}$-Alkyl und/oder O-$C_6$-$C_{10}$-Aryl)$_2$, Si($C_1$-$C_{12}$-Alkyl und/oder $C_6$-$C_{10}$-Aryl)$_2$(O-$C_1$-$C_{12}$-Alkyl oder O-$C_6$-$C_{10}$-Aryl), OS ($C_1$-$C_{12}$-Alkyl oder $C_6$-$C_{10}$-Aryl, $O_2$S($C_1$-$C_{12}$-Alkyl oder $C_6$-$C_{10}$-Aryl), CHO, CN, C(O-$C_1$-$C_{12}$-Alkyl oder -$C_6$-$C_{10}$-Aryl)$_2$, OC($C_1$-$C_{12}$-Alkyl oder $C_6$-$C_{10}$-Aryl), $NO_2$, $CF_3$, $OCF_3$, $OCF_2H$, $OCFH_2$, $OCF_2CF_3$, $OCH_2CF_3$, OCO ($C_1$-$C_{12}$-Alkyl oder $C_6$-$C_{10}$-Aryl), HNCO($C_1$-$C_{12}$-Alkyl oder $C_6$-$C_{10}$-Aryl)$_3$, OP($C_1$-$C_{12}$-Alkyl oder $C_6$-$C_{10}$-Aryl)$_3$, OP(O-$C_1$-$C_{10}$-Alkyl oder -$C_6$-$C_{10}$-Aryl)$_2$($C_1$-$C_{12}$-Alkyl oder $C_6$-$C_{10}$-Aryl) substituiert sein können

und/oder gegebenenfalls durch Sauerstoff- oder Schwefelatome oder N($C_1$-$C_{12}$-Alkyl oder $C_6$-$C_{10}$-Aryl)-Gruppen unterbrochen sein können,

wobei auch $R^1$ und $R^2$ oder $R^3$ und $R^4$ jeweils gemeinsam einen der zuvor definierten Reste, dann allerdings in zweibindiger Form darstellen können,

oder $R^3$ und $R^4$ gemeinsam einen 3- bis 10-gliedrigen Alkylring darstellen können, der gegebenenfalls mit einer bis zwei $C_1$-$C_{10}$-Alkylgruppen substituiert und/oder gegebenenfalls durch ein oder zwei Sauerstoff-, Schwefel- und/oder Stickstoffatomen unterbrochen sein kann,

A für Wasserstoff oder einen wie bei $R^1$ definierten Rest, jedoch in zweibindiger Form steht,

m für Null oder eine ganze Zahl von 1 bis 10 steht und

n für eine ganze Zahl von 1 bis 10 steht,

mit den Maßgaben, daß

a) $R^1$, $R^2$, $R^3$ und $R^4$ nicht für solche Reste stehen, die eine isolierte $\alpha,\beta$-Mehrfachbindung enthalten,

b) im Falle von $m \neq$ Null, wenigstens einer der Reste $R^1$, $R^2$, $R^3$ und $R^4$ für Wasserstoff steht,

c) im Falle A = Wasserstoff, m für Null steht und

d) im Falle A = Wasserstoff, $R^1$ und $R^2$ nicht gleichzeitig auch für Wasserstoff stehen.

**3.** Verfahren nach Anspruch 2, dadurch gekennzeichnet, daß man als basisch reagierende Amine solche der Formel (I) einsetzt, bei denen $R^1$, $R^2$, $R^3$ und $R^4$ gleich oder verschieden sind und jeweils für Wasserstoff oder einen der folgenden Reste in einbindiger Form stehen:

$C_1$-$C_{14}$-Alkyl, $C_3$-$C_{10}$-Alkenyl, $C_3$-$C_{10}$-Alkinyl, $C_3$-$C_{12}$-Cycloalkyl, $C_5$-$C_7$-Cycloalkenyl, $C_6$-$C_{10}$-Aryl, $C_5$-$C_{10}$-Hetaryl mit bis zu 3 Sauerstoff-, Schwefel- und/oder Stickstoffatomen im Ringsystem, $C_7$-$C_{12}$-Aralkyl oder $C_7$-$C_{10}$-Alkaryl,

die gegebenenfalls ein- bis fünfmal durch O-$C_1$-$C_6$-Alkyl oder -$C_6$-$C_{10}$-Aryl, $NH_2$, NH-$C_1$-$C_6$-Alkyl oder -$C_6$-$C_{10}$-Aryl, N($C_1$-$C_6$-Alkyl oder $C_6$-$C_{10}$-Aryl)$_2$, COO-$C_1$-$C_6$-Alkyl, COO-$C_6$-$C_{10}$-Aryl, CONH-$C_1$-$C_6$-Alkyl, CON ($C_1$-$C_6$-Alkyl)$_2$, OS($C_1$-$C_6$-Alkyl oder Phenyl), $O_2$S($C_1$-$C_6$-Alkyl oder Phenyl), CHO, CN, OC($C_1$-$C_6$-Alkyl oder Phenyl), $NO_2$, $CF_3$, $OCF_3$, $OCF_2H$, $OCFH_2$, OCO($C_1$-$C_6$-Alkyl oder Phenyl) oder HNCO($C_1$-$C_6$-Alkyl oder Phenyl substituiert sein können,

und/oder gegebenenfalls durch Sauerstoff- oder Schwefelatome oder N($C_1$-$C_6$-Alkyl oder Phenyl)-Gruppen unterbrochen sein können,

wobei auch $R^1$ und $R^2$ oder $R^3$ und $R^4$ jeweils gemeinsam einen der zuvor definierten Reste, dann allerdings in zweibindiger Form darstellen können,

oder $R^3$ und $R^4$ gemeinsam einen 5- bis 7-gliedrigen Alkylring darstellen können, der gegebenenfalls mit einer oder zwei $C_1$-$C_6$-Alkylgruppen substituiert ist und/oder gegebenenfalls durch ein Sauerstoff- oder Schwefelatom oder eine N($C_1$-$C_6$-Alkyl oder Phenyl)-Gruppe unterbrochen sein kann,

A für Wasserstoff oder einen der bei $R^1$ als bevorzugt definierten Reste in zweibindiger Form steht,

m für Null oder eine ganze Zahl von 1 bis 5 steht und

n für eine ganze Zahl von 1 bis 5 steht,

mit den Maßgaben, daß

a') $R^1$, $R^2$, $R^3$ und $R^4$ nicht für solche Reste stehen, die eine isolierte $\alpha,\beta$-Mehrfachbindung enthalten,

b') im Falle $m \neq$ Null wenigstens einer der Reste $R^1$, $R^2$, $R^3$ und $R^4$ für Wasserstoff steht,

c') im Falle A = Wasserstoff m für Null steht und

d') im Falle A = Wasserstoff $R^1$ und $R^2$ nicht gleichzeitig auch für Wasserstoff stehen.

**4.** Verfahren nach Ansprüchen 1 bis 3, dadurch gekennzeichnet, daß man als Kohlendioxid das übliche Handelsprodukt, Trockeneis oder mit Isotopen angereichertes Kohlendioxid einsetzt.

**5.** Verfahren nach Ansprüchen 1 bis 4, dadurch gekennzeichnet, daß man als Alkylierungsmittel Verbindungen der Formel (II) einsetzt

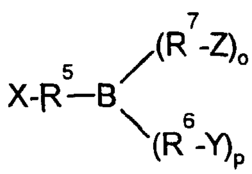

(II),

in der

$R^5$, $R^6$ und $R^7$ gleich oder verschieden sind und jeweils für einen der folgenden Reste in zweibindiger Form stehen: $C_1$-$C_{30}$-Alkyl, $C_4$-$C_{12}$-Cycloalkyl, $C_3$-$C_{30}$-Alkenyl, $C_5$-$C_{12}$-Cycloalkenyl, $C_3$-$C_{30}$-Alkinyl, $C_8$-$C_{12}$-Cycloalkinyl, $C_7$-$C_{20}$-Aralkyl, $C_8$-$C_{20}$-Aralkenyl, $C_8$-$C_{20}$-Aralkinyl, $C_8$-$C_{20}$-Alkenaryl, $C_8$-$C_{20}$-Alkinaryl, $C_5$-$C_{13}$-Alkenhetaryl oder $C_5$-$C_{13}$-Alkinhetaryl, die beiden letzten jeweils mit bis zu 3 Sauerstoff-, Schwefel- und/oder Stickstoffatomen im Ringsystem,

wobei kettenförmige Molekülteile linear, verzweigt und/oder gegebenenfalls durch Sauerstoff-, Schwefel-, N($C_1$-$C_{12}$-Alkyl oder $C_6$-$C_{10}$-Aryl)-, CO-, COO-, SO-, $SO_2$- oder SO(O)O- oder OP($C_1$-$C_{12}$-Alkyl oder $C_6$-$C_{10}$-Aryl)-Gruppen unterbrochen sein können,

wobei die Gruppierungen $R^6$-Y und $R^7$-Z auch für Wasserstoff stehen können und in diesem Falle $R^5$ gegebenenfalls zusätzlich durch COO($C_1$-$C_{12}$-Alkyl oder $C_6$-$C_{10}$-Aryl), CON($C_1$-$C_{12}$-Alkyl oder $C_6$-$C_{10}$-Aryl$)_2$, $C_6$-$C_{10}$-Aryl, O-C(O)-($C_1$-$C_{12}$-Alkyl oder $C_6$-$C_{10}$-Aryl), $CH_2$-O-(-C(O)-($C_1$-$C_{12}$-Alkyl oder $C_6$-$C_{10}$-Aryl), $C_5$-$C_{11}$-Hetaryl mit bis zu 3 Sauerstoff-, Schwefel- und/oder Stickstoffatomen im Ringsystem, O($C_1$-$C_{12}$-Alkyl oder $C_6$-$C_{10}$-Aryl), N($C_1$-$C_{12}$-Alkyl oder $C_6$-$C_{10}$-Aryl$)_2$, OP(O-$C_1$-$C_{12}$-Alkyl oder $C_6$-$C_{10}$-Aryl$)_2$, Si(O-$C_1$-$C_{12}$-Alkyl oder -$C_6$-$C_{10}$-Aryl$)_3$, Si($C_1$-$C_{12}$-Alkyl oder $C_6$-$C_{10}$-Aryl$)_3$ oder Halogen substituiert sein können,

o und p unabhängig voneinander jeweils für Null oder 1 stehen,

B nicht vorhanden ist, wenn o = p = Null ist,

B im Falle p = 1 und o = Null für einen der folgenden Reste in zweibindiger Form steht: $CH_2$, N($C_1$-$C_{12}$-Alkyl oder $C_6$-$C_{10}$-Aryl), OP($C_1$-$C_{12}$-Alkyl oder $C_6$-$C_{10}$-Aryl), Phenyl, Naphthyl, $(CH_2)_q$ mit q = 2 bis 30 oder $(CH_2)_r$-M-$(CH_2)_s$ mit r und s gleich oder verschieden und jeweils = 1 bis 20 und M = einem Sauerstoff- oder Schwefelatom oder einer $SO_2$- oder N($C_1$-$C_{12}$-Alkyl oder $C_6$-$C_{10}$-Aryl)-Gruppe,

B im Falle p = 1 und o = 1 für einen der folgenden Reste in dreibindiger Form steht: CH, N, (O)P, Phenyl oder Naphthyl und

X, Y und Z unabhängig voneinander für je eine Abgangsgruppe stehen.

**6.** Verfahren nach Ansprüchen 1 bis 5, dadurch gekennzeichnet, daß man als Alkylierungsmittel Verbindungen der Formel (III) einsetzt

$$X - R^{5'} \qquad (III),$$

in der
$R^{5'}$ für einen der folgenden Reste in einbindiger Form stehen: $C_1$-$C_{20}$-Alkyl, $C_3$-$C_{12}$-Cycloalkyl, $C_3$-$C_{30}$-Alkinyl, $C_7$-$C_{20}$-Aralkyl, $C_8$-$C_{20}$-Aralkenyl, $C_8$-$C_{20}$-Alkenaryl, $C_8$-$C_{20}$-Alkinaryl, $C_5$-$C_{13}$-Alkenhetaryl oder $C_5$-$C_{13}$-Alkinhetaryl, die beiden letzten jeweils mit bis zu 2 Sauerstoff-, Schwefel- und/oder Stickstoffatomen im Ringsystem und X für eine Abgangsgruppe steht.

**7.** Verfahren nach Ansprüchen 1 bis 6, dadurch gekennzeichnet, daß man pro eingesetztes Aminäquivalent 0,01 bis 10.000 Äquivalente Kohlendioxid und 0,01 bis 10.000 Äquivalente Alkylierungsmittel einsetzt.

**8.** Verfahren nach Ansprüchen 1 bis 7, dadurch gekennzeichnet, daß man als basische Verbindung der Elemente Lithium, Natrium, Magnesium, Kalium, Kalzium, Rubidium, Cäsium, Barium und/oder des Ammoniums Alkalicarbonate und/oder -hydrogencarbonate, insbesondere Kaliumcarbonat, einsetzt.

**9.** Verfahren nach Ansprüchen 1 bis 8, dadurch gekennzeichnet, daß man es in Gegenwart von Lösungsmitteln ausgewählt aus Kohlenwasserstoffen, Ethern, Aminen, Estern, Nitroverbindungen, Nitrilen, Tetrahydrothiophendioxid, Dimethylsulfoxid, Tetramethylensulfoxid, Propylsulfoxid, Benzylmethylsulfoxid, Diisobutylsulfoxid, Ketonen, verflüssigtem Kohlendioxid, Polyethernm des Ethylen- und/oder Propylenoxids und Amiden durchführt.

**10.** Verfahren nach Ansprüchen 1 bis 9, dadurch gekennzeichnet, daß man es in Gegenwart von Dimethylformamid oder Dimethylsulfoxid oder Gemischen von diesen mit anderen der im Anspruch 9 genannten Lösungsmittelndurchführt.

**11.** Verfahren nach Ansprüchen 1 bis 10, dadurch gekennzeichnet, daß man es bei -50 bis +180°C durchführt.

## Claims

**1.** Process for preparing organic carbamates from a basic amine, carbon dioxide and an alkylating agent, characterized in that the amine is first reacted with carbon dioxide in the presence of one or more basic compounds of the elements lithium, sodium, magnesium, potassium, calcium, rubidium, strontium, caesium, barium and/or of ammonium and the alkylating agent then added.

**2.** Process according to Claim 1, characterized in that the basic amines used are those of the formula (I)

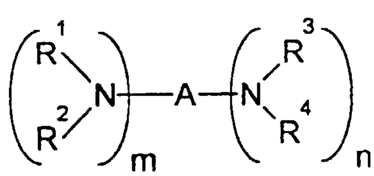

(I),

in which

$R^1$, $R^2$, $R^3$ and $R^4$ are the same or different and are each hydrogen or one of the following radicals in monovalent form: $C_1$-$C_{30}$-alkyl, $C_3$-$C_{30}$-alkenyl, $C_3$-$C_{30}$-alkinyl, $C_3$-$C_{12}$-cycloalkyl, $C_5$-$C_{12}$-cycloalkenyl, $C_8$-$C_{12}$-cycloalki-

nyl, $C_6$-$C_{14}$-aryl, $C_5$-$C_{13}$-hetaryl having up to 3 oxygen, sulphur and/or nitrogen atoms in the ring system, $C_7$-$C_{20}$-aralkyl, $C_9$-$C_{20}$-aralkenyl, $C_9$-$C_{20}$-aralkinyl, $C_7$-$C_{20}$-alkaryl, $C_8$-$C_{20}$-alkenaryl or $C_8$-$C_{20}$- alkinaryl,

which can optionally be substituted from one to five times by O-$C_1$-$C_{12}$-alkyl or -$C_6$-$C_{10}$-aryl, $NH_2$, NH-$C_1$-$C_{12}$-alkyl or -$C_6$-$C_{10}$-aryl, N($C_1$-$C_{12}$-alkyl or -$C_6$-$C_{10}$-aryl)$_2$, COO-$C_1$-$C_{12}$-alkyl or COO-$C_6$-$C_{10}$-aryl, $CONH_2$, CONH-$C_1$-$C_{12}$-alkyl, CON($C_1$-$C_{12}$-alkyl)$_2$, halogen, OP(O-$C_1$-$C_{12}$-alkyl or -$C_6$-$C_{10}$-aryl)$_2$, Si($C_1$-$C_{12}$-alkyl and/or $C_6$-$C_{10}$-aryl)$_3$, Si(O-$C_1$-$C_{12}$-alkyl and/or $C_6$-$C_{10}$-aryl)$_3$, Si($C_1$-$C_{12}$-alkyl or -$C_6$-$C_{10}$-aryl) (O-$C_1$-$C_{12}$-alkyl and/or O-$C_6$-$C_{10}$-aryl)$_2$, Si($C_1$-$C_{12}$-alkyl and/or $C_6$-$C_{10}$-aryl )$_2$(O-$C_1$-$C_{12}$-alkyl or O-$C_6$-$C_{10}$-aryl), OS($C_1$-$C_{12}$-alkyl or $C_6$-$C_{10}$-aryl), $O_2$S($C_1$-$C_{12}$-alkyl or $C_6$-$C_{10}$-aryl), CHO, CN, C(O-$C_1$-$C_{12}$-alkyl or -$C_6$-$C_{10}$-aryl)$_2$, OC($C_1$-$C_{12}$-alkyl or $C_6$-$C_{10}$-aryl), $NO_2$, $CF_3$, $OCF_3$, $OCF_2H$, $OCFH_2$, $OCF_2CF_3$, $OCH_2CF_3$, OCO($C_1$-$C_{12}$-alkyl or $C_6$-$C_{10}$-aryl), HNCO($C_1$-$C_{12}$-alkyl or $C_6$-$C_{10}$-aryl)$_3$, OP($C_1$-$C_{12}$-alkyl or $C_6$-$C_{10}$-aryl)$_3$, OP(O-$C_1$-$C_{10}$-alkyl or -$C_6$-$C_{10}$-aryl)$_2$($C_1$-$C_{12}$-alkyl or $C_6$-$C_{10}$-aryl)

and/or can optionally be interrupted by oxygen or sulphur atoms or by N($C_1$-$C_{12}$-alkyl or $C_6$-$C_{10}$-aryl) groups,

where $R^1$ and $R^2$ or $R^3$ and $R^4$ can, in each case together, also be one of the above-defined radicals, albeit then in divalent form,

or $R^3$ and $R^4$ can together be a from 3- to 10-membered alkyl ring, which can optionally be substituted with one or two $C_1$-$C_{10}$-alkyl groups and/or optionally be interrupted by one or two oxygen, sulphur and/or nitrogen atoms,

A is hydrogen or a radical as defined for $R^1$, although in divalent form,

m is zero or an integer from 1 to 10 and

n is an integer from 1 to 10,

with the provisos that

a) $R^1$, $R^2$, $R^3$ and $R^4$ are not radicals which contain an isolated $\alpha,\beta$ multiple bond,

b) in the case of m $\neq$ zero, at least one of the radicals $R^1$, $R^2$, $R^3$ and $R^4$ is hydrogen,

c) in the case of A = hydrogen, m is zero and

d) in the case of A = hydrogen, $R^1$ and $R^2$ are not also simultaneously hydrogen.

**3.** Process according to Claim 2, characterized in that the basic amines used are those of the formula (I) in which $R^1$, $R^2$, $R^3$ and $R^4$ are the same or different and are each hydrogen or one of the following radicals in monovalent form:

$C_1$-$C_{14}$-alkyl, $C_3$-$C_{10}$-alkenyl, $C_3$-$C_{10}$-alkinyl, $C_3$-$C_{12}$-cycloalkyl, $C_5$-$C_7$-cycloalkenyl, $C_6$-$C_{10}$-aryl, $C_5$-$C_{10}$-hetaryl having up to 3 oxygen, sulphur and/or nitrogen atoms in the ring system, $C_7$-$C_{12}$-aralkyl or $C_7$-$C_{10}$-alkaryl,

which can optionally be substituted one to five times by O-$C_1$-$C_6$-alkyl or -$C_6$-$C_{10}$-aryl, $NH_2$, NH-$C_1$-$C_6$-alkyl or -$C_6$-$C_{10}$-aryl, N($C_1$-$C_6$-alkyl or -$C_6$-$C_{10}$-aryl)$_2$, COO-$C_1$-$C_6$-alkyl, COO-$C_6$-$C_{10}$-aryl, CONH-$C_1$-$C_6$-alkyl, CON($C_1$-$C_6$-alkyl)$_2$, OS($C_1$-$C_6$)-alkyl or phenyl), $O_2$S($C_1$-$C_6$-alkyl or phenyl), CHO, CN, OC($C_1$-$C_6$-alkyl or phenyl), $NO_2$, $CF_3$, $OCF_3$, $OCF_2H$, $OCFH_2$, OCO-($C_1$-$C_6$-alkyl or phenyl) or HNCO($C_1$-$C_6$-alkyl or phenyl),

and/or can optionally be interrupted by oxygen or sulphur atoms or by N($C_1$-$C_6$-alkyl or phenyl) groups,

where $R^1$ and $R^2$ or $R^3$ and $R^4$ can, in each case together, also be one of the above-defined radicals, albeit then in divalent form,

or $R^3$ and $R^4$ can together be a from 5- to 7-membered alkyl ring, which can optionally be substituted with one or two $C_1$-$C_6$-alkyl groups and/or optionally be interrupted by an oxygen or sulphur atom or an N($C_1$-$C_6$-alkyl or phenyl) group,

A is hydrogen or a radical defined as preferred for $R^1$, in divalent form,

m is zero or an integer from 1 to 5 and

n is an integer from 1 to 5,

with the provisos that

a') $R^1$, $R^2$, $R^3$ and $R^4$ are not radicals which contain an isolated $\alpha,\beta$ multiple bond,

b') in the case of $m \neq$ zero, at least one of the radicals $R^1$, $R^2$, $R^3$ and $R^4$ is hydrogen,

c') in the case of A = hydrogen, m is zero and

d') in the case of A = hydrogen, $R^1$ and $R^2$ are not also simultaneously hydrogen.

**4.** Process according to Claims 1 to 3, characterized in that the carbon dioxide used is the usual commercial product, dry ice or isotopically enriched carbon dioxide.

**5.** Process according to Claims 1 to 4, characterized in that the alkylating agents used are compounds of the formula (II)

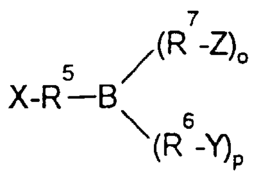

(II),

in which

$R^5$, $R^6$ and $R^7$ are the same or different and are each one of the following radicals in divalent form: $C_1$-$C_{30}$-alkyl, $C_4$-$C_{12}$-cycloalkyl, $C_3$-$C_{30}$-alkenyl, $C_5$-$C_{12}$-cycloalkenyl, $C_3$-$C_{30}$-alkinyl, $C_8$-$C_{12}$-cycloalkinyl, $C_7$-$C_{20}$-aralkyl, $C_8$-$C_{20}$-aralkenyl, $C_8$-$C_{20}$-aralkinyl, $C_8$-$C_{20}$-alkenaryl, $C_8$-$C_{20}$-alkinaryl, $C_5$-$C_{13}$-alkenehetaryl or C5-C13-alkinehetaryl, the latter two each having up to 3 oxygen, sulphur and/or nitrogen atoms in the ring system,

where chains in the molecules can be linear, branched and/or optionally interrupted by oxygen, sulphur, N($C_1$-$C_{12}$-or $C_6$-$C_{10}$-aryl), CO, COO, SO, $SO_2$ or SO(O)O or OP($C_1$-$C_{12}$-alkyl or $C_6$-$C_{10}$-aryl) groups,

where the groups $R^6$-Y and $R^7$-Z can also be hydrogen and in this case $R^5$ can optionally be additionally substituted by COO($C_1$-$C_{12}$-alkyl or $C_6$-$C_{10}$-aryl), CON($C_1$-$C_{12}$-alkyl or $C_6$-$C_{10}$-aryl)$_2$, $C_6$-$C_{10}$-aryl, O-C(O)-($C_1$-$C_{12}$-alkyl or $C_6$-$C_{10}$-aryl), $CH_2$-O-(-C(O)-($C_1$-$C_{12}$-alkyl or $C_6$-$C_{10}$-aryl), $C_5$-$C_{11}$-hetaryl having up to 3 oxygen, sulphur and/or nitrogen atoms in the ring system, O($C_1$-$C_{12}$-alkyl or $C_6$-$C_{10}$-aryl), N($C_1$-$C_{12}$-alkyl or $C_6$-$C_{10}$-aryl)$_2$, OP(O-$C_1$-$C_{12}$-alkyl or $C_6$-$C_{10}$-aryl)$_2$, Si(O-$C_1$-$C_{12}$-alkyl or -$C_6$-$C_{10}$-aryl)$_3$, Si($C_1$-$C_{12}$-alkyl or $C_6$-$C_{10}$-aryl)$_3$ or halogen,

o and p independently of one another are each zero or 1,

B is not present if o = p = zero,

in the case of p = 1 and o = zero, B is one of the following radicals in divalent form: $CH_2$, N($C_1$-$C_{12}$-alkyl or $C_6$-$C_{10}$-aryl), OP($C_1$-$C_{12}$-alkyl or $C_6$-$C_{10}$-aryl), phenyl, naphthyl, $(CH_2)_q$ with q = 2 to 30 or $(CH_2)_r$-M-$(CH_2)_s$ with r and s being the same or different and each = 1 to 20 and M = an oxygen or sulphur atom or an $SO_2$ or N($C_1$-$C_{12}$-alkyl or $C_6$-$C_{10}$-aryl) group,

in the case of p = 1 and o = 1, B is one of the following radicals in trivalent form: CH, N, (O)P, phenyl or naphthyl

and

X, Y and Z independently of one another are each a leaving group.

**6.** Process according to Claims 1 to 5, characterized in that the alkylating agents used are compounds of the formula (III)

$$X - R^{5'} \quad (III),$$

in which
$R^{5'}$ is one of the following radicals in monovalent form: $C_1$-$C_{20}$-alkyl, $C_4$-$C_{12}$-cycloalkyl, $C_3$-$C_{30}$-alkinyl, $C_7$-$C_{20}$-aralkyl, $C_8$-$C_{20}$-aralkenyl, $C_8$-$C_{20}$-alkenearyl, $C_8$-$C_{20}$-alkinearyl, $C_5$-$C_{13}$-alkenehetaryl or $C_5$-$C_{13}$-alkinehetaryl, the latter two each having up to 2 oxygen, sulphur and/or nitrogen atoms in the ring system and X is a leaving group.

**7.** Process according to Claims 1 to 6, characterized in that from 0.01 to 10,000 equivalents of carbon dioxide and from 0.01 to 10,000 equivalents of alkylating agent are used per equivalent of amine used.

**8.** Process according to Claims 1 to 7, characterized in that the basic compounds of the elements lithium, sodium, magnesium, potassium, calcium, rubidium, caesium, barium and/or of ammonium used are alkali metal carbonates and/or hydrogen carbonates, in particular calcium carbonate.

**9.** Process according to Claims 1 to 8, characterized in that it is carried out in the presence of solvents selected from hydrocarbons, ethers, amines, esters, nitro-compounds, nitriles, tetrahydrothiophene dioxide, dimethyl sulphoxide, tetramethylene sulphoxide, propyl sulphoxide, benzylmethyl sulphoxide, diisobutyl sulphoxide, ketones, liquefied carbon dioxide, polyethers of ethylene and/or propylene oxide and amides.

**10.** Process according to Claims 1 to 9, characterized in that it is carried out in the presence of dimethylformamide or dimethyl sulphoxide or mixtures of these with other solvents mentioned in Claim 9.

**11.** Process according to Claims 1 to 10, characterized in that it is carried out at from -50 to +180°C.

**Revendications**

**1.** Procédé pour la préparation de carbamates organiques à partir d'une amine à réaction basique, de dioxyde de carbone et d'un agent d'alkylation, caractérisé en ce qu'on amène d'abord à réagir l'amine avec le dioxyde de carbone en présence d'un ou de plusieurs composés basiques des éléments lithium, sodium, magnésium, potassium, calcium, rubidium, strontium, césium, baryum et/ou de l'ammonium, et on y ajoute ensuite l'agent d'alkylation.

**2.** Procédé selon la revendication 1, caractérisé en ce qu'on met en oeuvre, comme amines à réaction basique, celles répondant à la formule (I)

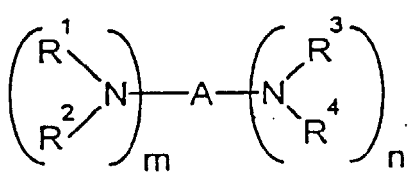

(I),

dans laquelle

$R^1$, $R^2$, $R^3$ et $R^4$ sont identiques ou différents et représentent respectivement un atome d'hydrogène ou un des radicaux ci-après sous la forme une fois liée: un groupe alkyle en $C_1$-$C_{30}$, un groupe alcényle en $C_3$-$C_{30}$, un groupe alcynyle en $C_3$-$C_{30}$, un groupe cycloalkyle en $C_3$-$C_{12}$, un groupe cycloalcényle en $C_5$-$C_{12}$, un groupe cycloalcynyle en $C_8$-$C_{12}$, un groupe aryle en $C_6$-$C_{14}$, un groupe hétaryle en $C_5$-$C_{13}$ contenant jusqu'à 3 atomes

d'oxygène, de soufre et/ou d'azote dans le système cyclique, un groupe aralkyle en $C_7$-$C_{20}$, un groupe aralcényle en $C_9$-$C_{20}$, un groupe aralcynyle en $C_9$-$C_{20}$, un groupe alkaryle en $C_7$-$C_{20}$, un groupe alcénaryle en $C_8$-$C_{20}$ ou un groupe alcynaryle en $C_8$-$C_{20}$,

qui, le cas échéant, peuvent porter de un à cinq substituants identiques ou différents O-alkyle en $C_1$-$C_{12}$ ou O-aryle en $C_6$-$C_{10}$, $NH_2$, NH-alkyle en $C_1$-$C_{12}$ ou NH-aryle en $C_6$-$C_{10}$, N(alkyle en $C_1$-$C_{12}$ ou aryle en $C_6$-$C_{10}$)$_2$, COO-alkyle en $C_1$-$C_{12}$ ou COO-aryle en $C_6$-$C_{10}$, $CONH_2$, CONH-alkyle en $C_1$-$C_{12}$, CON(alkyle en $C_1$-$C_{12}$)$_2$, halogéno, OP(O-alkyle en $C_1$-$C_{12}$ ou O-aryle en $C_6$-$C_{10}$)$_2$, Si(alkyle en $C_1$-$C_{12}$ et/ou aryle en $C_6$-$C_{10}$)$_3$, silo-alkyle en $C_1$-$C_{12}$ et/ou O-aryle en $C_6$-$C_{10}$)$_3$, Si(alkyle en $C_1$-$C_{12}$ ou aryle en $C_6$-$C_{10}$) (O-alkyle en $C_1$-$C_{12}$ et/ou O-aryle en $C_6$-$C_{10}$)$_2$, Si(alkyle en $C_1$-$C_{12}$ et/ou aryle en $C_6$-$C_{10}$)$_2$ (O-alkyle en $C_1$-$C_{12}$ ou O-aryle en $C_6$-$C_{10}$), OS(alkyle en $C_1$-$C_{12}$ ou aryle en $C_6$-$C_{10}$), $O_2$S(alkyle en $C_1$-$C_{12}$ ou aryle en $C_6$-$C_{10}$), CHO, CN, C(O-alkyle en $C_1$-$C_{12}$ ou O-aryle en $C_6$-$C_{10}$)$_2$, OC(alkyle en $C_1$-$C_{12}$ ou aryle en $C_6$-$C_{10}$), $NO_2$, $CF_3$, $OCF_3$, $OCF_2H$, $OCFH_2$, $OCF_2CF_3$, $OCH_2CF_3$, OCO(alkyle en $C_1$-$C_{12}$ ou aryle en $C_6$-$C_{10}$), HNCO(alkyle en $C_1$-$C_{12}$ ou aryle en $C_6$-$C_{10}$)$_3$, OP(alkyle en $C_1$-$C_{12}$ ou aryle en $C_6$-$C_{10}$)$_3$, OP(O-alkyle en $C_1$-$C_{10}$ ou O-aryle en $C_6$-$C_{10}$)$_2$(alkyle en $C_1$-$C_{12}$ ou aryle en $C_6$-$C_{10}$),

et/ou, le cas échéant, peuvent être interrompus par des atomes d'oxygène ou de soufre ou encore par des groupes N(alkyle en $C_1$-$C_{12}$ ou aryle en $C_6$-$C_{10}$),

dans laquelle également $R^1$ et $R^2$ ou $R^3$ et $R^4$ peuvent représenter respectivement ensemble un des radicaux définis ci-dessus, mais néanmoins sous la forme deux fois liée,

ou bien $R^3$ et $R^4$ peuvent représenter ensemble un noyau alkyle triangulaire à décagonal qui, le cas échéant, peut porter de 1 à 2 groupes alkyle en $C_1$-$C_{10}$ identiques ou différents et/ou, le cas échéant, peut être interrompu par un ou deux atomes d'oxygène, de soufre et/ou d'azote,

A représente un atome d'hydrogène ou un radical défini comme dans le cas de $R^1$, toutefois sous la forme deux fois liée,

m est égal à zéro ou représente un entier de 1 à 10, et

n représente un entier de 1 à 10,

avec les mesures que

a) $R^1$, $R^2$, $R^3$ et $R^4$ ne représentent pas des radicaux qui contiennent une liaison multiple $\alpha,\beta$ isolée,

b) dans le cas où m n'est pas égal à zéro, au moins un des radicaux $R^1$, $R^2$, $R^3$ et $R^4$ représente un atome d'hydrogène,

c) dans le cas où A représente un atome d'hydrogène, m représente zéro et

d) dans le cas où A représente un atome d'hydrogène, $R^1$ et $R^2$ ne représentent pas simultanément également un atome d'hydrogène.

**3.** Procédé selon la revendication 2, caractérisé en ce qu'on met en oeuvre, comme amines à réaction basique, celles répondant à la formule (I) dans laquelle $R^1$, $R^2$, $R^3$ et $R^4$ sont identiques ou différents et représentent respectivement un atome d'hydrogène ou un des radicaux ci-après sous la forme une fois liée:

un groupe alkyle en $C_1$-$C_{14}$, un groupe alcényle en $C_3$-$C_{10}$, un groupe alcynyle en $C_3$-$C_{10}$, un groupe cycloalkyle en $C_3$-$C_{12}$, un groupe cycloalcényle en $C_5$-$C_7$, un groupe aryle en $C_6$-$C_{10}$, un groupe hétaryle en $C_5$-$C_{10}$ contenant jusqu'à 3 atomes d'oxygène, de soufre et/ou d'azote dans le système cyclique, un groupe aralkyle en $C_7$-$C_{12}$ ou un groupe alkaryle en $C_7$-$C_{10}$,

qui, le cas échéant, peuvent porter de un à cinq substituants identiques ou différents O-alkyle en $C_1$-$C_6$ ou O-aryle en $C_6$-$C_{10}$, $NH_2$, NH-alkyle en $C_1$-$C_6$ ou NH-aryle en $C_6$-$C_{10}$, N(alkyle en $C_1$-$C_6$ ou aryle en $C_6$-$C_{10}$)$_2$, COO-alkyle en $C_1$-$C_6$, COO-aryle en $C_6$-$C_{10}$, CONHalkyle en $C_1$-$C_6$, CON(alkyle en $C_1$-$C_6$)$_2$, OS(alkyle en $C_1$-$C_6$ ou phényle), $O_2$S(alkyle en $C_1$-$C_6$ ou phényle), CHO, CN, OC(alkyle en $C_1$-$C_6$ ou phényle), $NO_2$, $CF_3$,

$OCF_3$, $OCF_2H$, $OCFH_2$, OCO(alkyle en $C_1$-$C_6$ ou phényle) ou encore HNCO(alkyle en $C_1$-$C_6$ ou phényle),

et/ou, le cas échéant, peuvent être interrompus par des atomes d'oxygène ou de soufre ou encore par des groupes N(alkyle en $C_1$-$C_6$ ou phényle),

dans laquelle également $R^1$ et $R^2$ ou $R^3$ et $R^4$ peuvent représenter respectivement ensemble un des radicaux définis ci-dessus, mais néanmoins sous la forme deux fois liée,

ou bien $R^3$ et $R^4$ peuvent représenter ensemble un noyau alkyle penta- à heptagonal qui, le cas échéant, porte un ou deux substituants alkyle en $C_1$-$C_6$ identiques ou différents et/ou, le cas échéant, peut être interrompu par un atome d'oxygène ou un atome de soufre ou encore par un groupe N(alkyle en $C_1$-$C_6$ ou phényle),

A représente un atome d'hydrogène ou un radical défini comme étant préféré dans le cas de $R^1$ sous la forme deux fois liée,

m est égal à zéro ou représente un entier de 1 à 5, et

n représente un entier de 1 à 5,

avec les mesures que

a') $R^1$, $R^2$, $R^3$ et $R^4$ ne représentent pas des radicaux qui contiennent une liaison multiple $\alpha,\beta$ isolée,

b') dans le cas où m n'est pas égal à zéro, au moins un des radicaux $R^1$, $R^2$, $R^3$ et $R^4$ représente un atome d'hydrogène,

c') dans le cas où A représente un atome d'hydrogène, m représente zéro et

d') dans le cas où A représente un atome d'hydrogène, $R^1$ et $R^2$ ne représentent pas simultanément également un atome d'hydrogène.

**4.** Procédé selon les revendications 1 à 3, caractérisé en ce qu'on met en oeuvre, comme dioxyde de carbone, le produit habituel disponible dans le commerce, de la glace carbonique ou du dioxyde de carbone enrichi avec des isotopes.

**5.** Procédé selon les revendications 1 à 4, caractérisé en ce qu'on met en oeuvre, comme agent d'alkylation, des composés répondant à la formule (II)

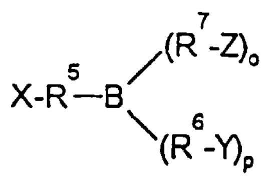

(II),

dans laquelle

$R^5$, $R^6$ et $R^7$ sont identiques ou différents et représentent respectivement un des radicaux ci-après sous la forme deux fois liée: un groupe alkyle en $C_1$-$C_{30}$, un groupe cycloalkyle en $C_4$-$C_{12}$, un groupe alcényle en $C_3$-$C_{30}$, un groupe cycloalcényle en $C_5$-$C_{12}$, un groupe alcynyle en $C_3$-$C_{30}$, un groupe cycloalcynyle en $C_8$-$C_{12}$, un groupe aralkyle en $C_7$-$C_{20}$, un groupe aralcényle en $C_8$-$C_{20}$, un groupe aralcynyle en $C_8$-$C_{20}$, un groupe alcénaryle en $C_8$-$C_{20}$, un groupe alcynaryle en $C_8$-$C_{20}$, un groupe alcène-hétaryle en $C_5$-$C_{13}$ ou un groupe alcyne-hétaryle en $C_5$-$C_{13}$, les deux derniers radicaux contenant jusqu'à 3 atomes d'oxygène, de soufre et/ou d'azote dans le système cyclique,

dans lequel des fractions moléculaires caténiformes peuvent être linéaires, ramifiées et/ou, le cas échéant, peuvent être interrompues par un atome d'oxygène, un atome de soufre, un groupe N(alkyle en $C_1$-$C_{12}$ ou

aryle en $C_6$-$C_{10}$), CO, COO, SO, $SO_2$ ou SO(O) ou encore un groupe OP(alkyle en $C_1$-$C_{12}$ ou aryle en $C_6$-$C_{10}$),

dans lequel les groupements $R^6$-Y et $R^7$-Z peuvent également représenter un atome d'hydrogène et, dans ce cas, $R^5$ peut, le cas échéant, porter en outre un ou plusieurs substituants identiques ou différents COO(alkyle en $C_1$-$C_{12}$ ou aryle en $C_6$-$C_{10}$), CON(alkyle en $C_1$-$C_{12}$ ou aryle en $C_6$-$C_{10}$)$_2$, aryle en $C_6$-$C_{10}$, O-C(O)- (alkyle en $C_1$-$C_{12}$ ou aryle en $C_6$-$C_{10}$), $CH_2$-O-(C(O)- (alkyle en $C_1$-$C_{12}$ ou aryle en $C_6$-$C_{10}$), hétaryle en $C_5$-$C_{11}$ contenant jusqu'à 3 atomes d'oxygène, de soufre ou d'azote dans le système cyclique, O(alkyle en $C_1$-$C_{12}$ ou aryle en $C_6$-$C_{10}$), N(alkyle en $C_1$-$C_{12}$ ou aryle en $C_6$-$C_{10}$)$_2$, OP(O-alkyle en $C_1$-$C_{12}$ ou O-aryle en $C_6$-$C_{10}$)$_2$, Si (O-alkyle en $C_1$-$C_{12}$ ou O-aryle en $C_6$-$C_{10}$)$_3$, Si(alkyle en $C_1$-$C_{12}$ ou aryle en $C_6$-$C_{10}$)$_3$ ou halogéno,

o et p représentent, indépendamment l'un de l'autre, respectivement zéro ou 1,

B n'est pas présent lorsque o = p = zéro,

B, dans le cas où p = 1 et o = zéro, représente un des radicaux ci-après sous la forme deux fois liée: $CH_2$, un groupe N(alkyle en $C_1$-$C_{12}$ ou aryle en $C_6$-$C_{10}$), un groupe OP(alkyle en $C_1$-$C_{12}$ ou aryle en $C_6$-$C_{10}$), un groupe phényle, un groupe naphtyle, $(CH_2)_q$ avec q = 2 à 30, ou encore un groupe $(CH_2)_r$-M-$(CH_2)_s$, r et s étant identiques ou différents et respectivement = 1 à 20, et M = un atome d'oxygène ou un atome de soufre ou encore un groupe $SO_2$ ou un groupe N(alkyle en $C_1$-$C_{12}$ ou aryle en $C_6$-$C_{10}$),

B, dans le cas où p = 1 et o = 1, représente un des radicaux ci-après sous la forme trois fois liée: CH, N, (O) P, un groupe phényle ou un groupe naphtyle et

X, Y et Z représentent, indépendamment l'un de l'autre, respectivement un groupe qui se sépare.

**6.** Procédé selon les revendications 1 à 5, caractérisé en ce qu'on met en oeuvre, comme agent d'alkylation, des composés de formule (III)

$$X\text{-}R^{5'} \quad \text{(III)}$$

dans laquelle
$R^{5'}$ représente un des radicaux ci-après sous la forme une fois liée: un groupe alkyle en $C_1$-$C_{20}$, un groupe cycloalkyle en $C_3$-$C_{12}$, un groupe alcynyle en $C_3$-$C_{30}$, un groupe aralkyle en $C_7$-$C_{20}$, un groupe aralcényle en $C_8$-$C_{20}$, un groupe alcénaryle en $C_8$-$C_{20}$, un groupe alcynaryle en $C_8$-$C_{20}$, un groupe alcène-hétaryle en $C_5$-$C_{13}$ ou un groupe alcyne-hétaryle en $C_5$-$C_{13}$, les deux derniers radicaux contenant respectivement jusqu'à 2 atomes d'oxygène, de soufre et/ou d'azote dans le système cyclique et X représente un groupe qui se sépare.

**7.** Procédé selon les revendications 1 à 6, caractérisé en ce que, par équivalent d'amine mis en oeuvre, on met en oeuvre de 0,01 à 10.000 équivalents de dioxyde de carbone et de 0,01 à 10.000 équivalents d'agent d'alkylation.

**8.** Procédé selon les revendications 1 à 7, caractérisé en ce qu'on met en oeuvre, comme composé basique des éléments lithium, sodium, magnésium, potassium, calcium, rubidium, césium, baryum et/ou de l'ammonium, des carbonates et/ou des hydrogénocarbonates de métaux alcalins, en particulier le carbonate de potassium.

**9.** Procédé selon les revendications 1 à 8, caractérisé en ce qu'on l'effectue en présence de solvants choisis parmi des hydrocarbures, des éthers, des amines, des esters, des composés nitro, des nitriles, le dioxyde de tétrahydrothiophène, le diméthylsulfoxyde, le tétraméthylènesulfoxyde, le propylsulfoxyde, le benzylméthylsulfoxyde, le diisobutylsulfoxyde, des cétones, du dioxyde de carbone liquéfié, des polyéthers de l'oxyde d'éthylène et/ou de l'oxyde de propylène et des amides.

**10.** Procédé selon les revendications 1 à 9, caractérisé en ce qu'on l'effectue en présence de diméthylformamide ou de diméthylsulfoxyde ou de mélanges de ces derniers avec d'autres solvants parmi ceux mentionnés à la revendication 9.

**11.** Procédé selon les revendications 1 à 10, caractérisé en ce qu'on le met en oeuvre à une température de -50 à +180°C.